(19) Europäisches Patentamt / European Patent Office / Office européen des brevets

(11) **EP 4 289 865 A1**

(12) **EUROPEAN PATENT APPLICATION**
published in accordance with Art. 153(4) EPC

(43) Date of publication:
**13.12.2023 Bulletin 2023/50**

(21) Application number: **22749125.5**

(22) Date of filing: **28.01.2022**

(51) International Patent Classification (IPC):
**C07K 16/28** (2006.01)     **C12N 15/13** (2006.01)
**A61K 39/395** (2006.01)     **A61K 47/68** (2017.01)
**A61P 35/00** (2006.01)

(52) Cooperative Patent Classification (CPC):
**A61K 39/395; A61K 47/68; A61P 35/00;
C07K 16/00; C07K 16/28**

(86) International application number:
**PCT/CN2022/074755**

(87) International publication number:
**WO 2022/166846 (11.08.2022 Gazette 2022/32)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **04.02.2021   CN 202110153466**

(71) Applicant: **Innovent Biologics (Suzhou) Co., Ltd.
Suzhou, Jiangsu 215123 (CN)**

(72) Inventors:
• **LI, Yiming
Suzhou, Jiangsu 215123 (CN)**
• **CHEN, Bingliang
Suzhou, Jiangsu 215123 (CN)**
• **JING, Hua
Suzhou, Jiangsu 215123 (CN)**

(74) Representative: **Sagittarius IP
Marlow International
Parkway
Marlow, SL7 1YL (GB)**

Remarks:
The complete document including Reference
Table(s) and the Sequence Listing(s) can be
downloaded from the EPO website

(54) **ANTI-TNFR2 ANTIBODY AND USE THEREOF**

(57)     The present invention relates to an anti-TNFR2 antibody, a preparation method therefor, a composition thereof, and use thereof. The present invention also provides a method for treating a TNFR2-associated disease and/or disorder, such as cancer.

EP 4 289 865 A1

**Description**

**TECHNICAL FIELD**

The present invention relates to an anti-TNFR2 antibody, a preparation method therefor, a composition thereof, and use thereof. The present invention also provides a method for treating a TNFR2-associated disease and/or disorder, such as cancer.

**BACKGROUND**

Type II tumor necrosis factor receptor (TNFR2, also known as CD120b or p75 or TNFRSF1B) is a member of the tumor necrosis factor (TNF) receptor superfamily. TNFR2, as a receptor for the cytokine TNFα, is highly expressed on the surface of various types of cancer cells, and regulatory T cells (Tregs) that can infiltrate into tumors to suppress immune system activity. TNFα, by binding to TNFR2, can activate downstream NFxB signaling, resulting in enhanced Treg cell proliferation. It has been found in various human and murine cancers that TNFR2-positive Treg cells were present in high abundance in the tumor microenvironment. Therefore, the treatment of cancer by targeting Treg cells in the tumor microenvironment and TNRF2 on tumor cells is a promising therapeutic regimen. For example, WO 2017/083525 discloses some antagonistic TNFR2 antibodies and proposes their use in the treatment of cancer.

In view of the potential target effect of TNFR2 in tumor immune responses, there is a need in the art to develop a novel TNFR2 antibody, particularly an anti-TNFR2 antibody with a strong blocking effect and direct tumor killing activity, for use in the treatment of a disease, particularly cancer.

**SUMMARY**

The inventors have provided a novel anti-TNFR2 antibody through intensive studies. The anti-TNFR2 antibodies of the present invention have strong TNFR2 blocking activity, and part of the molecules have a novel blocking mechanism, by which the antibodies can directly inhibit the polymerization of TNFR2 without blocking the binding of TNFα to a receptor TNFR2, thereby inhibiting the activation of the TNFα/TNFR2 signaling pathway. The anti-TNFR2 antibodies of the present invention exhibit good anti-tumor efficacy in animal experiments.

Therefore, in one aspect, the present invention provides an anti-TNFR2 antibody or an antigen-binding fragment thereof comprising: HCDR1, HCDR2, and HCDR3 sequences of one of heavy chain variable regions set forth in SEQ ID NOs: 19, 21, 23, 25, 27, 29, and 64, and/or LCDR1, LCDR2, and LCDR3 sequences of light chain variable regions set forth in SEQ ID NO: 20, 22, 24, 26, 28, 30, 31, or 65, or a variant of a combination of the CDR sequences. In some embodiments, the anti-TNFR2 antibody or the antigen-binding fragment thereof of the present invention has one or more of the following properties:

(i) binding to human TNFR-2 with high affinity, but not to human TNFR-1;
(ii) having cross immunoreactivity with monkey TNFR-2;
(iii) effectively binding to TNFR-2 on the surface of cells, especially tumor cells such as lymphoma cells and Treg cells;
(iv) blocking the binding of TNFR-2 to a ligand TNFα;
(v) inhibiting the activation of NF-xB signaling pathway mediated by the binding of TNFα to TNFR-2;
(vi) activating an ADCC signaling pathway; and
(vii) having anti-tumor activity, e.g., inhibiting tumor growth.

In some embodiments, the present invention also provides an anti-TNFR2 antibody or an antigen-binding fragment thereof. The antibody binds to the same or an overlapping epitope and/or competes for binding to TNFR2 with an exemplary antibody of the present invention (e.g., an antibody having a combination of VH and VL sequences of the antibody listed in Table B), and/or inhibits (e.g., competitively inhibits) the exemplary antibody of the present invention.

In some embodiments, the present invention provides a nucleic acid encoding the antibody or the antigen-binding fragment thereof of the present invention, a vector comprising the nucleic acid, and a host cell comprising the vector. In some embodiments, the present invention provides a method for preparing the antibody or the antigen-binding fragment thereof of the present invention.

In some embodiments, the present invention provides an immunoconjugate, a pharmaceutical composition, and a combination product comprising the antibody of the present invention.

In another aspect, the present invention provides *in vivo* and *in vitro* methods and use by using the TNFR2 antibody or the antigen-binding fragment thereof of the present invention for purposes including, for example, but not limited to, blocking and/or antagonizing the binding of TNFR-2 to its ligand TNFα, and/or blocking and/or antagonizing the bioactivity resulting from the binding, such as activation of a TNFR2 receptor and/or proliferation of Treg cells, *in*

*vivo* and *in vitro*; and/or killing tumor cells expressing TNFR2 on the surface *in vivo* and *in vitro*.

In yet another aspect, the present invention also provides a method and use for preventing or treating a TNFR2-associated disease, including, but not limited to, tumors, such as colon cancer or chronic myeloid leukemia. In some embodiments, the antibody of the present invention may be administered as the sole active agent or may be administered in combination with additional therapies or therapeutic agents.

In still another aspect, the present invention provides a method and a kit for detecting TNFR2 in a sample by using the antibody or the antigen-binding fragment thereof of the present invention.

The present invention is further illustrated in the following drawings and specific embodiments. However, these drawings and specific embodiments should not be construed as limiting the scope of the present invention, and modifications easily conceived by those skilled in the art will be included in the spirit of the present invention and the protection scope of the appended claims.

## BRIEF DESCRIPTION OF THE DRAWINGS

FIG. 1 shows an assay on the binding ability of hybridoma antibodies to a stable cell strain hTNFR2 CHO-S overexpressing human TNFR2 by flow cytometry.

FIG. 2 shows an assay on the binding ability of hybridoma antibodies to a stable cell strain cTNFR2 CHO-S expressing cynomolgus monkey TNFR2 by flow cytometry.

FIG. 3 shows an assay on the blocking effect of hybridoma antibodies on TNF$\alpha$/TNFR2 by ELISA.

FIG. 4 shows an assay on the binding ability of chimeric antibodies to a stable cell strain hTNFR2 CHO-S overexpressing human TNFR2 by flow cytometry.

FIG. 5 shows an assay on the binding ability of chimeric antibodies to a stable cell strain cTNFR2 CHO-S overexpressing cynomolgus monkey TNFR2 by flow cytometry.

FIG. 6 shows an assay on the blocking effect of chimeric antibodies on TNF$\alpha$/TNFR2 by ELISA.

FIG. 7 shows an assay on the inhibitory effect of chimeric antibodies on the TNF$\alpha$-activated TNFR2 signaling pathway by the Jurkat TNFR2 NF-$\kappa$B luciferase reporter system.

FIG. 8 shows an assay on the binding ability of humanized antibodies to a stable cell strain human hTNFR2 CHO-S by flow cytometry.

FIG. 9 shows an assay on the blocking effect of humanized antibodies on TNF$\alpha$/TNFR2 by ELISA.

FIG. 10 shows an assay on the inhibitory effect of humanized antibodies on the TNF$\alpha$-activated TNFR2 signaling pathway by the Jurkat TNFR2 NF-$\kappa$B luciferase reporter system.

FIG. 11 shows an assay on the binding ability of humanized antibodies to a human K562 CML cell strain (A) and primary Treg cells (B) by flow cytometry.

FIG. 12 shows an assay on the activation of the ADCC signaling pathway by humanized antibodies by the Jurkat ADCC luciferase reporter system.

FIG. 13 shows an assay on the anti-tumor effect of TNFR2 antibodies using a K562 matrigel NOD/SCID animal model.

FIG. 14 shows an assay on the anti-tumor effect of TNFR2 antibodies using a C57 animal model subcutaneously inoculated with MC38-TNFR2.

FIG. 15 shows CDR sequences of exemplary chimeric antibodies and humanized antibodies of the present invention.

FIG. 16 shows VH and VL sequences of exemplary chimeric antibodies and humanized antibodies of the present invention.

## DETAILED DESCRIPTION

Definitions

Unless otherwise defined, all technical and scientific terms used herein have the same meaning as those commonly understood by those of ordinary skill in the art. For the purposes of the present invention, the following terms are defined below.

The term "about" used in combination with a numerical value is intended to encompass the numerical values in a range from a lower limit less than the specified numerical value by 5% to an upper limit greater than the specified numerical value by 5%.

The term "and/or" should be understood to refer to any one of the options or any two or more of the options.

As used herein, the term "comprise" or "include" is intended to mean that the elements, integers, or steps are included, but not to the exclusion of any other elements, integers, or steps. The term "comprise" or "include" used herein, unless indicated otherwise, also encompasses the situation where the entirety consists of the described elements, integers, or steps. For example, when referring to an antibody variable region "comprising" a specific sequence, it is also intended to encompass an antibody variable region consisting of the specific sequence.

As used herein, the term "antibody" refers to a polypeptide comprising at least an immunoglobulin light chain variable region or heavy chain variable region that specifically recognizes and binds to an antigen. The term "antibody" encompasses a variety of antibody structures, including but not limited to, monoclonal antibodies, polyclonal antibodies, single-chain or multi-chain antibodies, monospecific or multispecific antibodies (e.g., bispecific antibodies), fully human or chimeric or humanized antibodies, and full-length antibodies and antibody fragments, so long as they exhibit the desired antigen-binding activity.

As will be clear to those skilled in the art, a "whole antibody" (used interchangeably herein with "full-length antibody", "complete antibody", and "intact antibody") comprises at least two heavy chains (Hs) and two light chains (Ls). Each heavy chain consists of a heavy chain variable region (abbreviated herein as VH) and a heavy chain constant region. Each heavy chain constant region consists of 3 domains, i.e., CH1, CH2, and CH3. Each light chain consists of a light chain variable region (abbreviated herein as VL) and a light chain constant region. Each light chain constant region consists of one domain CL. The variable regions are domains in the heavy chains or light chains of an antibody that participate in the binding of the antibody to an antigen thereof. The constant regions are not directly involved in the binding of the antibody to the antigen but exhibit a variety of effector functions. The light chains of an antibody can be assigned to one of two types, kappa ($\kappa$) and lambda ($\lambda$), based on the amino acid sequence of a constant domain thereof. Depending on the amino acid sequence of the heavy chain constant region, the heavy chains of an antibody can be divided into 5 major types, i.e., IgA, IgD, IgE, IgG, and IgM, several of which can be further divided into subtypes, e.g., IgG1, IgG2, IgG3, IgG4, IgA1, and IgA2. Heavy chain constant regions corresponding to different antibody types are called $\alpha$, $\delta$, $\epsilon$, y, and $\mu$, respectively. The term "isotype" refers to an antibody type determined by the heavy chain constant regions of the antibody. See, e.g., Fundamental Immunology, Ch.7 (Paul, w. eds., 2nd edition, Raven Press, N.Y. (1989)) which is incorporated herein by reference in its entirety for all purposes.

The term "antigen-binding moiety" (used interchangeably herein with "antibody fragment" and "antigen-binding fragment") of the antibody refers to an incomplete antibody molecule that comprises a portion of an intact antibody for binding to an antigen to which the intact antibody binds. As will be understood by those skilled in the art, the antigen-binding moiety of an antibody generally comprises amino acid residues from a "complementarity determining region" or a "CDR". The antigen-binding fragment may be prepared by a recombinant DNA technique, or by enzymatic or chemical cleavage of an intact antibody. The antigen-binding fragment includes, but is not limited to, a Fab, an scFab, a Fab', an F(ab')$_2$, a Fab'-SH, an Fv, a single-chain Fv, a diabody, a triabody, a tetrabody, a minibody, and a single-domain antibody (sdAb). For more detailed descriptions of the antibody fragment, see: Fundamental Immunology, W.E. Paul eds., Raven Press, N.Y. (1993); Shao Rongguang et al. (eds.), Antibody Drug Research and Application, People's Medical Publishing House (2013); Hollinger et al., PNAS USA 90: 6444-6448 (1993); Hudson et al., Nat. Med. 9: 129-134 (2003).

The term "chimeric antibody" refers to an antibody in which the variable region sequences are derived from one species and the constant region sequences are derived from another species, e.g., an antibody in which the variable region sequences are derived from a mouse antibody and the constant region sequences are derived from a human antibody.

The term "humanized antibody" refers to an antibody in which CDR sequences derived from another mammalian species, such as mice, are linked to human framework sequences. Additional framework region modifications may be made within the human framework sequences and/or additional amino acid modifications may be made in the CDR sequences, for example, to perform affinity maturation of the antibody.

An "isolated" antibody is an antibody that has been separated from components in its natural environment. In some embodiments, the antibody is purified to a purity greater than 95% or 99% as determined by, for example, electrophoresis (e.g., SDS-PAGE, isoelectric focusing (IEF), capillary electrophoresis), or chromatography (e.g., ion exchange or reverse-phase HPLC). For a review of methods for assessing antibody purity, see, e.g., Flatman, S. et al., J. Chrom. B 848 (2007) 79-87.

The term "epitope" is an antigen region to which an antibody binds. Epitopes can be formed by contiguous amino acids or noncontiguous amino acids juxtaposed by tertiary folding of a protein.

Herein, TNRF2 refers to "tumor necrosis factor receptor 2", also known as TNFRSF1B and CD120b. The receptor is a member of the tumor necrosis factor receptor superfamily (TNFRSF), and as a cell surface membrane-bound receptor, can be expressed on cancer cells and tumor-infiltrating Treg cells. TNFR2 is capable of regulating the transcription of genes that promote cell survival and proliferation by modulating the NF-$\kappa$B signaling pathway. Herein, the expression encompasses TNFR2 and variants, isoforms, homologs, and species homologs thereof. In one embodiment according to the present invention, TNFR2 is human-derived TNFR2, e.g., human TNFR2 set forth in SEQ ID NO: 60, or human TNFR2 having at least 95%, even at least 96%, 97%, 98%, or 99% amino acid sequence identity to SEQ ID NO: 60. In another embodiment according to the present invention, TNFR2 is monkey-derived TNFR2, e.g., human TNFR2 set forth in SEQ ID NO: 61, or human TNFR2 having at least 95%, even at least 96%, 97%, 98%, or 99% amino acid sequence identity to SEQ ID NO: 61. Herein, TNFR2 may also include fragments of TNFR2, such as fragments comprising an extracellular domain, e.g., fragments that retain the ability to bind to any antibody of the present invention. Herein, TNFα refers to a natural agonist ligand "tumor necrosis factor α" of TNFR2. Herein, TNFR1 refers to "tumor necrosis factor receptor 1", e.g., human TNFR1 under UniProtKB accession number P19438.

The term "specifically binds to" means that an antibody selectively or preferentially binds to an antigen. If an antibody binds to human TNFR2 with a $K_D$ of about $5 \times 10^{-7}$ M or less, about $1 \times 10^{-7}$ M or less, about $5 \times 10^{-8}$ M or less, about $1 \times 10^{-8}$ M or less, or about $5 \times 10^{-9}$ M or less as measured by biological optical interferometry, the antibody is an antibody that "specifically binds to human TNFR2". However, the antibody that specifically binds to human TNFR2 may have cross-reactivity with a TNFR2 protein from other species. For example, an antibody specific to human TNFR2, in some embodiments, can cross-react with a TNFR2 protein from a non-human species. In some other embodiments, an antibody specific to human TNFR2 may be completely specific to human TNFR2 while not exhibiting cross-reactivity to other species, or exhibiting cross-reactivity only to TNFR2 from certain species.

As used herein, the term "cross-reactivity" refers to the ability of an antibody to bind to TNFR2 from different species. For example, the antibody binding to human TNFR2 described herein can also bind to TNFR2 from other species (e.g., cynomolgus monkey TNFR2). A method for determining cross-reactivity includes the method described in examples and standard assays known in the art, such as biological optical interferometry or flow cytometry.

"Affinity" or "binding affinity" refers to inherent binding affinity that reflects interactions between members of a binding pair. The affinity of a molecule X for its partner Y can be generally represented by an equilibrium dissociation constant ($K_D$), which is the ratio of a dissociation rate constant ($k_{dis}$) to an association rate constant ($k_{on}$). Affinity can be measured by common methods known in the art. One specific method for measuring affinity is the ForteBio kinetic binding assay described herein.

For an IgG antibody, the term "high affinity" means that the antibody binds to a target antigen with a $K_D$ of $1 \times 10^{-7}$ M or less, preferably $5 \times 10^{-8}$ M or less, more preferably about $1 \times 10^{-8}$ M or less, and even more preferably about $5 \times 10^{-9}$ M or less. However, "high affinity" binding may vary with antibody isotypes. For example, for IgM isotypes, "high affinity" means that an antibody has a $K_D$ of $1 \times 10^{-6}$ M or less, preferably $1 \times 10^{-7}$ M or less, and more preferably about $1 \times 10^{-8}$ M or less.

An "antibody that competes for binding" to an antigen (e.g., TNFR2) with a reference antibody is an antibody that blocks the binding of the reference antibody to the antigen (e.g., TNFR2) by 50% or more in a competitive assay, and conversely, the reference antibody blocks the binding of the antibody to the antigen (e.g., TNFR2) by 50% or more in the competitive assay. Exemplary competitive assays are described in: "Antibodies", Harbor and Lane (Cold Spring Harbor Press, Cold Spring Harbor, NY). The antibody that competes for binding and the reference antibody can bind to the same epitope region, e.g., the same epitope, adjacent epitopes, or overlapping epitopes.

An antibody that inhibits (e.g., competitively inhibits) the binding of a reference antibody to its antigen refers to an antibody that inhibits the binding of the reference antibody to its antigen by 50%, 60%, 70%, 80%, 90%, or 95% or more. Conversely, the reference antibody inhibits the binding of the antibody to its antigen by 50%, 60%, 70%, 80%, 90%, or 95% or more. The binding of an antibody to its antigen can be measured by affinity (e.g., equilibrium dissociation constant). Methods for determining affinity are known in the art.

An antibody that shows identical or similar binding affinity and/or specificity as a reference antibody refers to an antibody that is capable of having at least 50%, 60%, 70%, 80%, 90%, or 95% or more of the binding affinity and/or specificity of the reference antibody. This can be determined by any method known in the art for determining binding affinity and/or specificity.

The term "Fc region" is used herein to define the C-terminal region of an immunoglobulin heavy chain that comprises at least a part of the constant regions. The term includes Fc regions of native sequences and variant Fc regions. In one embodiment, a human IgG heavy chain Fc region extends from Cys226 or from Pro230 of a heavy chain to the carboxyl terminus. However, the C-terminal lysine (Lys447) of the Fc region may or may not be present. Unless otherwise indicated herein, amino acid residues in the Fc region or constant regions are numbered according to the EU numbering system, also referred to as the EU index, as described in Kabat, E.A. et al., Sequences of Proteins of Immunological Interest, 5th Ed., Public Health Service, National Institutes of Health, Bethesda, MD (1991), NIH Publication 91-3242.

The term "effector function" refers to those bioactivities attributable to the antibody Fc region that varies with the class of antibody. There are five known major classes of antibodies: IgA, IgD, IgE, IgG, and IgM, some of which can be further divided into subclasses (isotypes), e.g., IgG1, IgG2, IgG3, IgG4, IgA1, and IgA2. The IgG Fc region can mediate several important effector functions, e.g., cytokine induction, ADCC, phagocytosis, complement-dependent cytotoxicity (CDC), and half-life/clearance rate of an antibody and an antigen-antibody complex. In some cases, depending on the therapeutic purpose, these effector functions may be desirable for a therapeutic antibody, but in some other cases, they may be unnecessary or even detrimental. Therefore, in one embodiment, the present invention provides a variant Fc region having amino acid residue changes in the Fc region that change antibody effector functions, and a screening method therefor. For example, at least one amino acid residue in the Fc region of an antibody may be replaced, thereby changing the effector functions of the antibody.

"ADCC" refers to antibody-dependent cell-mediated cytotoxicity. ADCC is mediated in humans primarily by natural killer cells (NK cells). In ADCC, an antibody binds to an antigen displayed on the surface of a target cell, and FcγRIIIA on the surface of NK cells recognizes the Fc region of the antibody, so that NK cells are activated to release perforin and granzyme, resulting in lysis and apoptosis of the target cell. Non-limiting examples of *in vitro* assays for assessing the ADCC activity of a target molecule are described in US 5,500,362 (or see, e.g., Hellstrom, I. et al., Proc. Nαt'l Acαd. Sci. USA 83 (1986) 7059-7063; Hellstrom, I. et al., Proc. Nαt'l Acαd. Sci. USA 82 (1985) 1499-1502); and US 5,821,337 (or see Bruggemann, M. et al., J. Exp. Med. 166 (1987) 1351-1361). Alternatively, non-radioactive assays (e.g., ACTITM non-radioactive cytotoxicity assays (CellTechnology, Inc. Mountain View, CA) and CytoTox96® non-radioactive cytotoxicity assays (Promega, Madison, WI) for flow cytometry) may be employed. Effector cells suitable for use in these assays include peripheral blood mononuclear cells (PBMC) and natural killer (NK) cells. Alternatively or additionally, the ADCC activity of the target molecule may be assessed *in vivo,* for example, in an animal model as disclosed in Clynes, R. et al., Proc. Nαt'l Acαd. Sci. USA 95 (1998) 652-656.

"CDC" refers to complement-dependent cytotoxicity. In CDC, the Fc region of an antibody binds to the complement molecule C1q to form a membrane attack complex, resulting in clearance of the target cell. See, e.g., Liszewski and Atkinson, ch. 26, Fundamental immunology, 3rd edition, Paul eds., Raven Press, New York, 1993, pp 917-940.

"ADCP" refers to antibody-dependent cell-mediated phagocytosis. In this process mediated by the Fc receptor, target cells bound by the antibody are phagocytosed by phagocytic cells such as macrophages, monocytes, neutrophils, and dendritic cells. A variety of Fc receptors can be involved in this process. *In vitro* experiments for ADCP are described in Richards et al., Mol. Cancer Ther. 7(8): 2517-2527 (2008).

The term "variant" herein related to an antibody refers to an antibody that comprises a target antibody region having amino acid changes by virtue of at least one, for example, 1-30, 1-20, or 1-10, e.g., 1, 2, 3, 4, or 5 amino acid substitutions, deletions, and/or insertions, when compared to the reference antibody, wherein the variant substantially retains at least one biological property (e.g., antigen-binding ability) of the antibody molecule prior to change. The target antibody region may be the full length of the antibody, or the heavy chain variable region or the light chain variable region or a combination thereof, or one or more heavy chain CDR regions or one or more light chain CDR regions or a combination thereof. Herein, an antibody region having amino acid changes relative to a reference antibody region is also referred to as a "variant" of the reference antibody region.

Herein, "sequence identity" refers to the degree to which sequences are identical on a nucleotide-by-nucleotide or amino acid-by-amino acid basis in a comparison window. The "percent sequence identity" can be calculated by the following steps: comparing two optimally aligned sequences in a comparison window; determining the number of positions in which nucleic acid bases (e.g., A, T, C, G, and I) or amino acid residues (e.g., Ala, Pro, Ser, Thr, Gly, Val, Leu, Ile, Phe, Tyr, Trp, Lys, Arg, His, Asp, Glu, Asn, Gln, Cys, and Met) are identical in the two sequences to yield the number of matched positions; dividing the number of matched positions by the total number of positions in the comparison window (i.e., the window size); and multiplying the result by 100 to yield the percent sequence identity. Optimal alignment for determining the percent sequence identity may be achieved in a variety of ways known in the art, for example, using publicly available computer software such as BLAST, BLAST-2, ALIGN, or Megalign (DNASTAR) software. Those skilled in the art can determine suitable parameters for alignment of the sequences, including any algorithm necessary to yield optimal alignment within a full-length sequence range or target sequence region being compared.

Herein, with respect to antibody sequences, the percent amino acid sequence identity is determined by optimally aligning a candidate antibody sequence with a reference antibody sequence, and in a preferred embodiment, optimal alignment is performed according to the Kabat numbering scheme. Herein, without specifying the comparison window (i.e., the target antibody region to be compared), it will be applicable to align over the full length of the reference antibody sequence. In some embodiments, with respect to antibodies, the sequence identity may be achieved throughout the heavy chain variable region and/or the light chain variable region, or the percent sequence identity may be limited to the framework regions only, while the sequences of corresponding CDR regions remain 100% identical.

Similarly, with respect to antibody sequences, a candidate antibody having amino acid changes in the target antibody region relative to a reference antibody can be determined based on the alignment.

Herein, "conservative substitution" refers to an amino acid change that results in the replacement of an amino acid with a chemically similar amino acid. Amino acid modifications such as substitutions can be introduced into the antibody of the invention by standard methods known in the art, such as site-directed mutagenesis and PCR-mediated mutagenesis.

Conservative replacement tables that provide functionally similar amino acids are well known in the art. In a preferred aspect, conservatively substituted residues are from the Table A of conservative substitutions below, preferably are the preferred conservatively substituted residues shown in Table A.

Table A

| Original residue | Exemplary substitution | Preferred conservative substitution |
|---|---|---|
| Ala (A) | Val; Leu; Ile | Val |
| Arg (R) | Lys; Gln; Asn | Lys |
| Asn (N) | Gln; His; Asp; Lys; Arg | Gin |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gin; Lys; Arg | Arg |
| Ile (I) | Leu; Val; Met; Ala; Phe; Nle | Leu |
| Leu (L) | Nle; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Nle | Leu |

All aspects of the present invention are further detailed in the following sections.

**I. Anti-TNFR2 Antibody of the Present Invention**

In one aspect, the present invention provides an antibody or an antigen-binding fragment thereof, particularly a humanized antibody or antigen-binding fragment thereof, that specifically binds to TNFR2, preferably a human TNFR2 protein (e.g., the human TNFR2 sequence of SEQ ID NO: 60, NM-001066.2). In some embodiments, the antigen-binding fragment of the antibody of the present invention is an antibody fragment selected from: a Fab, a Fab', a Fab'-SH, an Fv, a single-chain antibody such as an scFv, an $(Fab')_2$ fragment, a single-domain antibody, a diabody (dAb), and a linear antibody.

Advantageous biological properties of antibodies

In some embodiments, the anti-TNFR2 antibody or the antigen-binding fragment thereof of the present invention binds to human TNFR2 with high affinity, e.g., with a dissociation equilibrium constant ($K_D$) of less than or equal to about $50 \times 10^{-9}$ M, more preferably about $1\text{-}30 \times 10^{-9}$ M, and more preferably less than or equal to about $5 \times 10^{-9}$ M, e.g., about 2 nM, 1.5 nM, 1.0 nM, 0.5 nM, 0.3 nM, or 0.2 nM. Preferably, $K_D$ is determined by biological optical interferometry (e.g., Fortebio affinity assay). In some embodiments, the anti-TNFR2 antibody or the antigen-binding fragment thereof of the present invention binds to human TNFR2 with a dissociation constant of about $1\text{-}20 \times 10^{-4}$ $s^{-1}$, e.g., about $2 \times 10^{-4}$ $s^{-1}$, about $5 \times 10^{-4}$ $s^{-1}$, about $10 \times 10^{-4}$ $s^{-1}$, about $15 \times 10^{-4}$ $s^{-1}$, about $20 \times 10^{-4}$ $s^{-1}$, e.g., as determined by a Fortebio affinity assay.
In some embodiments, the anti-TNFR2 antibody or the antigen-binding fragment thereof of the present invention cross-reacts with monkey TNFR2. In some embodiments, the antibody binds to cynomolgus monkey TNFR2 with high affinity, wherein the $K_D$ value (e.g., as measured by a Fortebio affinity assay) is less than or equal to about $50 \times 10^{-9}$ M, more preferably about $1\text{-}30 \times 10^{-9}$ M, and more preferably less than or equal to about $5 \times 10^{-9}$ M.
In some embodiments, the antibody or the antigen-binding fragment thereof of the present invention binds to

7

TNFR2 expressed on the cell surface with high affinity. In one embodiment, the cell expressing human TNFR2 on the surface is a tumor cell (e.g., a lymphoma cell, such as a K562 cell), a Treg cell (e.g., a human peripheral blood native $CD4^+$ $CD25^+$ $CD127^{LOW}$ regulatory T cell), or a mammalian cell recombinantly expressing TNFR2. Preferably, the $EC_{50}$ value for the binding of the antibody to cells expressing human TNFR2 is determined by flow cytometry (e.g., FACS). In some embodiments, the $EC_{50}$ value for the binding of the antibody of the present invention to TNFR2 on the cell surface is less than 10 nM, e.g., 0.1-5 nM, e.g., as measured by flow cytometry on CHO cells recombinantly expressing TNFR2. In some other embodiments, the $EC_{50}$ value for the binding of the antibody of the present invention to TNFR2 on the cell surface is less than 100 pM, e.g., 20-60 pM, e.g., as measured by flow cytometry on K562 cells or human peripheral blood Treg cells. As will be clear to those skilled in the art, in some cases, the density of cell surface TNFR2 will affect the $EC_{50}$ measurement for the binding of the antibody to cells.

In some embodiments, the antibody or the antigen-binding fragment thereof of the present invention blocks the binding of TNFR2 to its ligand TNFα. The ability of an antibody to block the binding of human TNFR2 (TNFR2 expressed on cells) to human TNFα (e.g., IC50 value and maximum blocking activity) can be determined by flow cytometry (e.g., FACS). Preferably, the antibody of the present invention is capable of completely blocking the binding of human TNFR2 (TNFR2 expressed on cells) to human TNFα. In some embodiments, the $IC_{50}$ value for the antibody of the present invention is less than about 10 nM, e.g., about 1 nM, 2 nM, 3 nM, 4 nM, 5 nM, or 6nM, as measured by FACS.

In some embodiments, the antibody or the antigen-binding fragment thereof of the present invention reduces the activation of NF-xB signaling pathway caused by the binding of TNFα to TNFR2. In some embodiments, the ability of an antibody to reduce the activation of NF-xB signaling pathway caused by the binding of TNFα to TNFR2 (e.g., maximal inhibition and IC50 value) is determined by a fluorescent reporter gene assay (e.g., the Jurkat NF-κB luciferase reporter assay of the examples). In some embodiments, the antibody blocks the activation of NF-κB signaling pathway caused by the binding of TNFα to TNFR2, achieving three-fold or more inhibition as compared to no antibody addition. In some embodiments, the $IC_{50}$ value for the antibody is less than about 0.1-10 nM.

In some embodiments, the binding of the antibody or the antigen-binding fragment thereof of the present invention to TNFR2 on cells activates the ADCC signaling pathway in the presence of NK and/or effector T cells. Preferably, the antibody of the present invention can kill TNFR2-positive tumor cells by ADCC activity. In some embodiments, the ability of an antibody to elicit an ADCC fluorescent signal by binding to TNFR2 (e.g., maximal activation and $EC_{50}$ value) is determined by a fluorescent reporter assay (e.g., the Jurkat ADCC luciferase reporter assay of the examples).

In some embodiments, the antibody or the antigen-binding fragment thereof of the present invention inhibits the growth of a tumor expressing human TNFR2. In one embodiment, the tumor cell is a lymphoma cell expressing human TNFR2, such as a chronic myeloid leukemia cell, or a solid tumor cell expressing human TNFR2, such as a colon cancer cell. In yet another embodiment, the antibody or the antigen-binding fragment thereof of the present invention elicits ADCC-dependent tumor cell-killing activity. In some other embodiments, the antibody or the antigen-binding fragment thereof of the present invention inhibits the function of immunosuppressive regulatory Treg cells expressing TNFR2 (e.g., in a tumor microenvironment).

In some embodiments, the antibody or the antigen-binding fragment thereof of the present invention has at least one or preferably both of the following effects:

- acting on immunosuppressive Treg cells to inhibit the proliferation of the Treg cells, thereby facilitating the immune function recovery of a host; and
- directly acting on cancer cells expressing TNFR2 on the surface and eliciting ADCC-dependent tumor cell-killing activity.

In some embodiments, the antibody or the antigen-binding fragment thereof of the present invention achieves at least 10% tumor growth inhibition, e.g., at least 20%-50% or greater tumor growth inhibition, in a subject (e.g., a tumor-bearing animal model, such as the tumor-bearing mouse of the examples).

Preferably, the antibody or the antigen-binding fragment thereof of the present invention exhibits at least one, preferably at least two, more preferably at least three, four, or five, and even more preferably all of the above properties.

CDR regions of antibodies

"Complementarity determining region", "CDR region", or "CDR" (used interchangeably herein with a hypervariable region "HVR") is an amino acid region in a variable region of an antibody that is primarily responsible for binding to an epitope. The CDRs of the heavy and light chains are generally referred to as CDR1, CDR2, and CDR3, and are numbered sequentially from the N-terminus. The CDRs located in the heavy chain variable domain of the antibody are referred to as HCDR1, HCDR2, and HCDR3, whereas the CDRs located in the light chain variable domain of the antibody are referred to as LCDR1, LCDR2, and LCDR3.

Combinations of the VH and VL sequences of some exemplary antibodies of the present invention are listed in Table B below:

| Antib ody | VH, which comprises or consists of an amino acid sequence set forth in SEQ ID NO as follows | VL, which comprises or consists of an amino acid sequence set forth in SEQ ID NO as follows |
| --- | --- | --- |
| 1 | SEQ ID NO:19 | SEQ ID NO:20 |
| 2 | SEQ ID NO:21 | SEQ ID NO:22 |
| 3 | SEQ ID NO:23 | SEQ ID NO:24 |
| 4 | SEQ ID NO:25 | SEQ ID NO:26 |
| 5 | SEQ ID NO:27 | SEQ ID NO:28 |
| 6 | SEQ ID NO:29 | SEQ ID NO:30 |
| 7 | SEQ ID NO:29 | SEQ ID NO:31 |
| 8 | SEQ ID NO:64 | SEQ ID NO:65 |

Various schemes for determining the CDR sequence of a given VH or VL amino acid sequence are known in the art. For example, Kabat complementarity determining regions (CDRs) are determined based on sequence variability and are the most commonly used (Kabat et al., Sequences of Proteins of Immunological Interest, 5th Ed. Public Health Service, National Institutes of Health, Bethesda, Md. (1991)). Chothia scheme is based on the positions of structural loops (Chothia and Lesk, J. Mol. Biol. 196: 901-917 (1987)). AbM HVRs are a compromise between Kabat HVRs and Chothia structural loops and are used by AbM antibody modeling software from Oxford Molecular. "Contact" HVRs are based on an analysis of available complex crystal structures. According to different CDR determination schemes, the residue of each HVR/CDR among these HVRs is described as follows.

| CDR | Kabat scheme | AbM scheme | Chothia scheme | Contact scheme |
| --- | --- | --- | --- | --- |
| LCDR1 | L24-L34 | L24-L34 | L26-L32 | L30-L36 |
| LCDR2 | L50-L56 | L50-L56 | L50-L52 | L46-L55 |
| LCDR3 | L89-L97 | L89-L97 | L91-L96 | L89-L96 |
| HCDR1 | H31-H35B | H26-H35B | H26-H32 | H30-H35B |
| (Kabat numbering system) | | | | |
| HCDR1 | H31-H35 | H26-H35 | H26-H32 | H30-H35 |
| (Chothia numbering system) | | | | |
| HCDR2 | H50-H65 | H50-H58 | H53-H55 | H47-H58 |
| HCDR3 | H95-H102 | H95-H102 | H96-H101 | H93-H101 |
| (Kabat numbering system) | | | | |

HVRs may also be HVR sequences located at the following Kabat residue positions according to the Kabat numbering system:
positions 24-36 or 24-34 (LCDR1), positions 46-56 or 50-56 (LCDR2), and positions 89-97 or 89-96 (LCDR3) in the VL; and positions 26-35 or 27-35B (HCDR1), positions 50-65 or 49-65 (HCDR2), and positions 93-102, 94-102, or 95-102 (HCDR3) in the VH.

HVRs may also be determined based on the same Kabat numbering positions of a reference CDR sequence (e.g., any one of the exemplary CDRs of the present invention).

Unless otherwise stated, the term "CDR", "CDR sequence", "HVR", or "HVR sequence" used herein encompasses HVR or CDR sequences determined in any of the ways described above.

Unless otherwise stated, residue positions of an antibody variable region (including heavy chain variable region residues and light chain variable region residues) are numbered according to the Kabat numbering system (Kabat et al., Sequences of Proteins of Immunological Interest, 5th Ed. Public Health Service, National Institutes of Health, Bethesda, Md. (1991)).

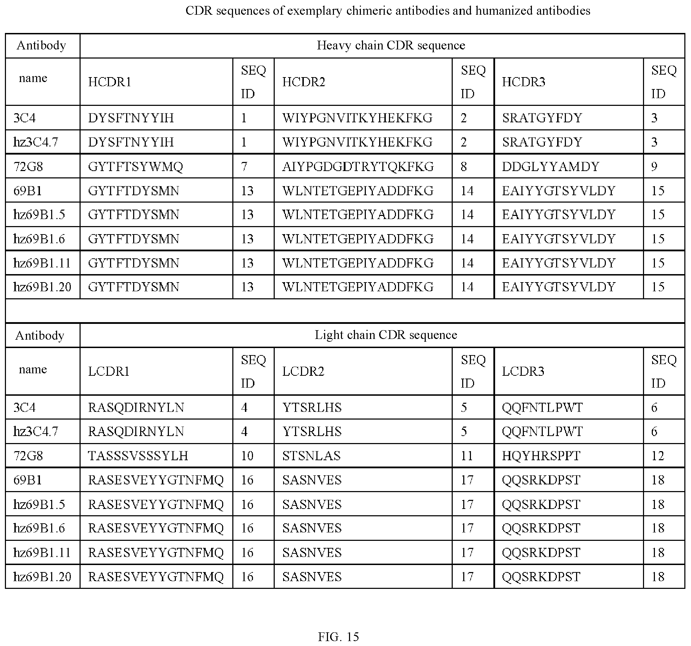

In another preferred embodiment, the CDR sequences of the present invention are shown in FIG. 15.

Combinations of some exemplary CDR sequences of the present invention are listed in Table C below:

| Combination | HCDR1, which comprises or consists of an amino acid sequence set forth in SEQ ID NO as follows | HCDR2, which comprises or consists of an amino acid sequence set forth in SEQ ID NO as follows | HCDR3, which comprises or consists of an amino acid sequence set forth in SEQ ID NO as follows | LCDR1, which comprises or consists of an amino acid sequence set forth in SEQ ID NO as follows | LCDR2, which comprises or consists of an amino acid sequence set forth in SEQ ID NO as follows | LCDR3, which comprises or consists of an amino acid sequence set forth in SEQ ID NO as follows |
|---|---|---|---|---|---|---|
| | | | | | | |
| 1 | SEQ ID NO:1 | SEQ ID NO:2 | SEQ ID NO: 3 | SEQ ID NON | SEQ ID NO: 5 | SEQ ID NON |
| 2 | SEQ ID NO:7 | SEQ ID NO: 8 | SEQ ID NO: 9 | SEQ ID NO: 10 | SEQ ID NO: 11 | SEQ ID NO: 12 |
| 3 | SEQ ID NO: 13 | SEQ ID NO: 14 | SEQ ID NO: 15 | SEQ ID NO: 16 | SEQ ID NO: 17 | SEQ ID NO: 18 |

Antibodies with different specificities (i.e., different binding sites for different antigens) have different CDRs. However, although CDRs vary from antibody to antibody, only a limited number of amino acid positions within the CDRs are directly involved in antigen binding. The smallest overlapping region can be determined using at least two of the Kabat, Chothia, AbM, and Contact schemes, thereby providing a "minimal binding unit" for antigen binding. The minimal binding unit may be a sub-portion of the CDR. As will be clear to those skilled in the art, residues in the remaining portions of the CDR sequences can be determined by the structure and protein folding of the antibody. Therefore, variants of any CDR presented herein are also considered. For example, in a variant of one CDR, the amino acid residue of the minimal binding unit may remain unchanged, while the remaining CDR residues defined by the Kabat or Chothia may be conservatively substituted.

In some embodiments, the antibody of the present invention has at least one, two, three, four, five, or six CDRs that are identical to, or are variants of, corresponding CDRs in the variable region sequences of any of the antibodies listed in Table B. In some embodiments, the antibody of the present invention has at least one, two, or three HCDRs that are identical to, or are variants of, corresponding heavy chain CDRs in the variable region sequences of any of the antibodies listed in Table B. In some embodiments, the antibody of the present invention has at least one, two, or three LCDRs that are identical to, or are variants of, corresponding light chain CDRs in the variable region sequences of any of the antibodies listed in Table B. Herein, "corresponding CDRs" refer to CDRs that are located at positions in the amino acid sequences of a variable region of a candidate antibody that are most similar to where CDRs of a reference antibody locate at after optimal alignment. Herein, a CDR variant is a CDR that has been modified by at least one, e.g., 1 or 2 or 3 amino acid substitutions, deletions, and/or insertions, wherein an antigen-binding molecule comprising the CDR variant substantially retains the biological properties of the antigen-binding molecule comprising the unmodified CDR, e.g., retains at least 60%, 70%, 80%, 90%, or 100% of the bioactivity (e.g., antigen-binding ability). It can be understood that the CDRs may be modified alone or in combination. Preferably, an amino acid modification is an amino acid substitution, in particular a conservative amino acid substitution, such as a preferred conservative amino acid replacement listed in Table A.

In addition, it is known in the art that the CDR3 region, which is independent of the CDR1 and/or CDR2 regions, alone can determine the binding specificity of an antibody to an associated antigen. Furthermore, a variety of other antibodies having the same binding specificity can be generated based on the consensus CDR3 sequence.

Thus, in one embodiment, the antibody of the present invention comprises a CDR3 sequence from the heavy and/or light chain variable region sequence of any one of the antibodies shown in Table B, wherein the antibody is capable of specifically binding to human TNFR2. In yet another embodiment, the antibody may further comprise CDR2 from the heavy and/or light chain variable region of the same antibody, or CDR2 from the heavy and/or light chain variable region of different TNFR2 antibodies. In yet another embodiment, the antibody may further comprise CDR1 from the heavy and/or light chain variable region of the same antibody, or CDR1 from the heavy and/or light chain variable region of different TNFR2 antibodies. Activities of these antibodies, including an activity of binding to human TNFR2, an activity of blocking the binding of TNFR2 to a TNFα molecule, and/or an activity of inhibiting tumor growth, can be characterized by assays described herein.

In yet another aspect, given that the antigen-binding specificity is dependent primarily on the CDR1, CDR2, and CDR3 regions, in some embodiments, VH CDR1, CDR2, and CDR3 sequences and VL CDR1, CDR2, and CDR3 sequences can be "combined and paired" (i.e., CDRs from different antibodies that bind to the same TNFR2 antigen can be combined and paired, and each antibody preferably comprises VH CDR1, CDR2, and CDR3 and VL CDR1, CDR2, and CDR3) to produce other molecules of the present invention that bind to TNFR2. The binding of such "combined and paired" antibodies to TNFR2 can be tested by binding assays known in the art (e.g., ELISA, SET, and Biacore) and other assays described in the examples. When VH CDR sequences are combined and paired, CDR1, CDR2, and/or CDR3 sequences from a particular VH sequence are preferably substituted with structurally similar CDR sequences. Likewise, when VL CDR sequences are combined and paired, CDR1, CDR2, and/or CDR3 sequences from a particular VL sequence are preferably substituted with structurally similar CDR sequences. CDRs can be "combined and paired" among the antibodies shown in Table B of the present invention. In addition, as will be clear to those skilled in the art that the other antibodies of the present invention may also be generated by replacing the structurally similar CDR sequences of the antibodies of the present invention with one or more of VH CDR and/or VL CDR sequences from other different antibodies.

Therefore, in some embodiments, the antibody or the antigen-binding fragment thereof of the present invention comprises a heavy chain variable region comprising a heavy chain complementarity determining region 3 (HCDR3), wherein the HCDR3:

(i) is identical to HCDR3 of the heavy chain variable region of any one of the antibodies listed in Table B; or
(ii) is identical to any one of the HCDR3 sequences listed in Table C; or
(iii) comprises at least 1 (preferably 1-2, and more preferably 1) amino acid change (preferably substitution, and more preferably conservative substitution) relative to the HCDR3 of (i) or (ii).

In some embodiments, the antibody or the antigen-binding fragment thereof of the present invention comprises a heavy chain variable region and a light chain variable region, wherein the heavy chain complementarity determining regions 3 (HCDR3) and light chain complementarity determining regions 3 (LCDR3) of the antibody:

(i) are identical to HCDR3 and LCDR3 of the heavy and light chain variable region sequences of any one of the antibodies listed in Table B; or
(ii) are identical to HCDR3 and LCDR3 sequences in any one of the combinations listed in Table C; or
(iii) comprise at least 1 (preferably 1-2, and more preferably 1) amino acid change (preferably substitution, and more preferably conservative substitution) in total relative to the HCDR3 and LCDR3 of (i) or (ii).

In one embodiment, the antibody or the antigen-binding fragment thereof of the present invention comprises a heavy chain variable region (VH), wherein the VH comprises:

(i) HCDR1, HCDR2, and HCDR3 sequences comprised in a VH sequence of any one of the antibodies listed in Table B; or
(ii) HCDR1, HCDR2, and HCDR3 sequences in any one of the combinations listed in Table C; or
(iii) sequences having at least one and no more than 5, 4, 3, 2, or 1 amino acid change (preferably amino acid substitution, preferably conservative substitution) in the three CDR regions in total relative to the sequences of (i) or (ii).

In another embodiment, the antibody or the antigen-binding fragment thereof of the present invention comprises a light chain variable region (VL), wherein the VL comprises:

(i) LCDR1, LCDR2, and LCDR3 sequences comprised in a VL sequence of any one of the antibodies listed in Table B; or
(ii) LCDR1, LCDR2, and LCDR3 sequences in any one of the combinations listed in Table C; or
(iii) sequences having at least one and no more than 5, 4, 3, 2, or 1 amino acid change (preferably amino acid substitution, preferably conservative substitution) in the three CDR regions in total relative to the sequences of (i) or (ii).

In another embodiment, the antibody or the antigen-binding fragment thereof of the present invention comprises a heavy chain variable region and a light chain variable region, wherein the antibody comprises:

(i) six CDR sequences comprised in VH and VL sequences of any one of the antibodies listed in Table B; or
(ii) six CDR sequences in any one of the combinations listed in Table C; or
(iii) sequences having at least one and no more than 10, 5, 4, 3, 2, or 1 amino acid change (preferably amino acid substitution, preferably conservative substitution) in the six CDR regions in total relative to the sequences of (i) or (ii).

In one embodiment, the antibody or antigen-binding fragment thereof of the present invention comprises:

(i) HCDR1, HCDR2, and HCDR3 sequences of a heavy chain variable region set forth in SEQ ID NO: 19, and LCDR1, LCDR2, and LCDR3 sequences of a light chain variable region set forth in SEQ ID NO: 20, or
(ii) HCDR1, HCDR2, and HCDR3 sequences of a heavy chain variable region set forth in SEQ ID NO: 21 and LCDR1, LCDR2, and LCDR3 sequences of a light chain variable region set forth in SEQ ID NO: 22, or
(iii) HCDR1, HCDR2, and HCDR3 sequences of a heavy chain variable region set forth in SEQ ID NO: 23 and LCDR1, LCDR2, and LCDR3 sequences of a light chain variable region set forth in SEQ ID NO:24, or
(iv) HCDR1, HCDR2, and HCDR3 sequences of a heavy chain variable region set forth in SEQ ID NO: 25 and LCDR1, LCDR2, and LCDR3 sequences of a light chain variable region set forth in SEQ ID NO: 26, or
(v) HCDR1, HCDR2, and HCDR3 sequences of a heavy chain variable region set forth in SEQ ID NO: 27 and LCDR1, LCDR2, and LCDR3 sequences of a light chain variable region set forth in SEQ ID NO: 28, or
(vi) HCDR1, HCDR2, and HCDR3 sequences of a heavy chain variable region set forth in SEQ ID NO:29 and LCDR1, LCDR2, and LCDR3 sequences of a light chain variable region set forth in SEQ ID NO: 30 or 31, or
(vii) HCDR1, HCDR2, and HCDR3 sequences of a heavy chain variable region set forth in SEQ ID NO: 64, and LCDR1, LCDR2, and LCDR3 sequences of a light chain variable region set forth in SEQ ID NO: 65.

In a preferred embodiment, the antibody or the antigen-binding fragment thereof of the present invention comprises three complementarity determining regions of a heavy chain variable region (HCDRs), and three complementarity determining regions of a light chain variable region (LCDRs), wherein

(i) HCDR1 comprises or consists of an amino acid sequence of SEQ ID NO: 1, HCDR2 comprises or consists of an amino acid sequence of SEQ ID NO: 2, HCDR3 comprises or consists of an amino acid sequence of SEQ ID NO: 3, LCDR1 comprises or consists of an amino acid sequence of SEQ ID NO: 4, LCDR2 comprises or consists of an amino acid sequence of SEQ ID NO: 5, and LCDR3 comprises or consists of an amino acid sequence of SEQ ID NO: 6; or
(ii) HCDR1 comprises or consists of an amino acid sequence of SEQ ID NO: 7, HCDR2 comprises or consists of an amino acid sequence of SEQ ID NO: 8, HCDR3 comprises or consists of an amino acid sequence of SEQ ID NO: 9, LCDR1 comprises or consists of an amino acid sequence of SEQ ID NO: 10, LCDR2 comprises or consists of an amino acid sequence of SEQ ID NO: 11, and LCDR3 comprises or consists of an amino acid sequence of SEQ ID NO: 12; or
(iii) HCDR1 comprises or consists of an amino acid sequence of SEQ ID NO: 13, HCDR2 comprises or consists of an amino acid sequence of SEQ ID NO: 14, HCDR3 comprises or consists of an amino acid sequence of SEQ ID NO: 15, LCDR1 comprises or consists of an amino acid sequence of SEQ ID NO: 16, LCDR2 comprises or consists of an amino acid sequence of SEQ ID NO: 17, and LCDR3 comprises or consists of an amino acid sequence of SEQ ID NO: 18.

In one preferred embodiment, the antibody or the antigen-binding fragment thereof of the present invention comprises 6 CDR sequences of one of the combinations listed in Table C.

Variable regions of antibodies

A "variable region" or "variable domain" is a domain in the heavy or light chain of an antibody that participates in the binding of the antibody to the antigen thereof. A heavy chain variable region (VH) and a light chain variable region (VL) can be further subdivided into hypervariable regions (HVRs, also known as complementarity determining regions (CDRs)) with more conservative regions (i.e., framework regions (FRs)) inserted therebetween. Each VH or VL consists of three CDRs and four FRs, arranged from the amino terminus to the carboxyl terminus in the following order: FR1, CDR1, FR2, CDR2, FR3, CDR3, and FR4. In some cases, a single VH or VL domain is sufficient to provide the antigen-binding specificity. Furthermore, an antibody binding to a particular antigen can be isolated by screening libraries of complementarity VL or VH domains by virtue of VH or VL domains from an antibody binding to the antigen (see, e.g., Portolano, S. et al., J. Immunol. 150 (1993) 880-887; Clackson, T. et al., Nature 352 (1991) 624-628).

It is known in the art that one or more residues in one or both of the variable regions (i.e., VH and/or VL) can be modified, for example, one or more CDR regions and/or one or more framework regions undergo residue modifications, particularly conservative residue substitutions, and the modified antibody still substantially retains at least one biological property (e.g., antigen-binding ability) of the antibody molecule prior to the modification. For example, residues in CDR regions may be mutated to improve one or more binding properties (e.g., affinity) of the antibody. The antigen-binding properties or other functional properties of the mutated antibody can be assessed in an *in vitro* or *in vivo* assay. Preferably, conservative substitutions are introduced. Preferably, no more than 1, 2, 3, 4, or 5 residue modifications are introduced in the CDR regions. Furthermore, residues in framework regions can be mutated, for example, to improve the properties of the antibody. For example, one or more residues in the framework regions may be "back mutated" to corresponding residues of a germline sequence.

CDR grafting is another modification method for antibody variable region known in the art. Since CDR sequences are responsible for most of the antibody-antigen interactions, a recombinant antibody variant that simulates the properties of known antibodies can be constructed. In the antibody variant, CDR sequences from the known antibodies are grafted onto framework regions of different antibodies having different properties. Therefore, in one embodiment, the present invention relates to an anti-TNFR2 antibody or an antigen-binding fragment thereof, wherein the antibody comprises CDR sequences of the heavy and light chain variable regions from one of the antibodies of Table B, and has different framework region sequences. A framework region sequence for substitution can be obtained from a public DNA database, including germline antibody gene sequences, or from published TNFR2 antibody sequences. For example, germline DNAs encoding human heavy and light chain variable region genes can be obtained from the GenBank database. Antibody protein sequences can be compared to protein sequences in the database using sequence similarity search tools, such as Gapped BLAST. Preferably, the framework region sequence for substitution is structurally similar to a framework sequence of the antibody of the present invention selected for change, e.g., a framework sequence having at least 80%, 85%, 90%, 95%, 96%, 97%, 98%, 99% or more sequence identity. In some embodiments, humanization of the antibody may be performed according to the method of Example 9.

In yet another embodiment, VH and VL sequences from an exemplary antibody of the present invention (one of the antibodies shown in Table B) and other different anti-TNFR2 antibodies (preferably, another antibody shown in Table B) can be "combined and paired" to produce other antibodies of the present invention binding to TNFR2. When these chains are combined and paired, it is preferred that the VH sequence from a particular VH/VL pair is replaced with a structurally similar VH sequence. Likewise, the VL sequence from a particular VH/VL pair is preferably replaced with a structurally similar VL sequence. The binding of such "combined and paired" antibodies to TNFR2 can be tested by binding assays known in the art (e.g., ELISA, and other assays described in the examples).

Therefore, in one embodiment, the antibody of the present invention comprises or consists of a heavy chain variable region (VH) sequence of any one of the antibodies listed in Table B. In yet another embodiment, the antibody of the present invention comprises a variant of the VH sequence.

In another embodiment, the antibody of the present invention comprises or consists of a light chain variable region (VL) sequence of any one of the antibodies listed in Table B. In yet another embodiment, the antibody of the present invention comprises a variant of the VL sequence.

In yet another embodiment, the antibody of the present invention comprises:

(i) a VH sequence comprising an amino acid sequence set forth in SEQ ID NO: 19 or a variant thereof, and/or a VL sequence comprising an amino acid sequence set forth in SEQ ID NO: 20 or a variant thereof, or

(ii) a VH sequence comprising an amino acid sequence set forth in SEQ ID NO: 21 or a variant thereof, and/or a VL sequence comprising an amino acid sequence set forth in SEQ ID NO: 22 or a variant thereof, or

(iii) a VH sequence comprising an amino acid sequence set forth in SEQ ID NO: 23 or a variant thereof, and/or a VL sequence comprising an amino acid sequence set forth in SEQ ID NO: 24 or a variant thereof, or

(iv) a VH sequence comprising an amino acid sequence set forth in SEQ ID NO: 25 or a variant thereof, and/or a VL sequence comprising an amino acid sequence set forth in SEQ ID NO: 26 or a variant thereof, or

(v) a VH sequence comprising an amino acid sequence set forth in SEQ ID NO: 27 or a variant thereof, and/or a VL sequence comprising an amino acid sequence set forth in SEQ ID NO: 28 or a variant thereof, or

(vi) a VH sequence comprising an amino acid sequence set forth in SEQ ID NO: 29 or a variant thereof, and/or a VL sequence comprising an amino acid sequence set forth in SEQ ID NO: 30 or 21 or a variant thereof, or

(vii) a VH sequence comprising an amino acid sequence set forth in SEQ ID NO: 64 or a variant thereof, and/or a VL sequence comprising an amino acid sequence set forth in SEQ ID NO: 65 or a variant thereof.

In one embodiment, with respect to the amino acid sequence, a variant of the VH sequence has at least 80%, 85%, 90%, 92%, 95%, 97%, 98%, 99%, or more identity relative to the reference VH sequence (preferably, in terms of the full length, or the three regions of CDR1, CDR2, and CDR3). In one embodiment, with respect to the amino acid sequence, a variant of the VH sequence comprises at least one and no more than 30, 10, 5, 4, 3, 2, 1, or 0 amino acid changes (preferably amino acid substitutions, and more preferably conservative substitutions) relative to the reference VH sequence (preferably, in terms of the full length, or the three regions of CDR1, CDR2, and CDR3). Preferably, sequence differences do not occur in the CDR regions.

In a preferred embodiment, with respect to the amino acid sequence, a variant of the VL sequence has at least 80%, 85%, 90%, 92%, 95%, 97%, 98%, 99%, or more identity relative to the reference VL sequence (preferably, in terms of the full length, or the three regions of CDR1, CDR2, and CDR3). In a preferred embodiment, with respect to the amino acid sequence, a variant of the VL sequence comprises at least one and no more than 30, 10, 5, 4, 3, 2, 1, or 0 amino acid changes (preferably amino acid substitutions, and more preferably conservative substitutions) relative to the reference VL sequence (preferably, in terms of the full length, or the three regions of CDR1, CDR2, and CDR3). Preferably, sequence differences do not occur in the CDR regions.

In a preferred embodiment, the antibody of the present invention comprises or consists of a VH/VL sequence pair of the heavy and light chain variable regions of any one of the antibodies listed in Table B. The present invention also provides a variant of the antibody, e.g., a variant having at least 95%-99% identity or comprising no more than 10 amino acid changes in VH, VL, or VH and VL.

In any one of the above embodiments, preferably, in terms of one or more CDR regions (preferably all three CDR regions), a heavy chain variable region of an antibody variant comprises no more than 10, preferably no more than 5 (e.g., 3, 2, 1, or 0), amino acid changes (preferably amino acid substitutions, and more preferably conservative substitutions) relative to a reference antibody.

In any one of the above embodiments, preferably, in terms of one or more CDR regions (preferably all three CDR regions), a light chain variable region of an antibody variant comprises no more than 10, preferably no more than 5 (e.g., 3, 2, 1, or 0), amino acid changes (preferably amino acid substitutions, and more preferably conservative substitutions) relative to a reference antibody.

Heavy and light chains of antibodies

In one embodiment, the antibody of the present invention comprises a heavy chain constant region and/or a light chain constant region. In some embodiments, the antibody of the present invention comprises a heavy chain Fc region, e.g., an Fc region of the IgG1, IgG2, IgG3, or IgG4 isotype. In some other embodiments, the antibody of the present invention comprises an IgG1 Fc region, particularly a human IgG1 Fc region. In still some other embodiments, the antibody of the present invention comprises a κ light chain constant region, e.g., a human κ light chain constant region.

Depending on the desired application of the antibody, the antibody may comprise a heavy chain constant region having the same or changed effector function relative to the native Fc region. Exemplary "effector functions" include C1q binding, complement-dependent cytotoxicity (CDC), Fc receptor binding, and FcγR-mediated effector functions such as ADCC and antibody-dependent cell-mediated phagocytosis (ADCP). In general, effector functions are elicited when the variable region of an antibody binds to an antigen on the surface of a cell (e.g., a tumor cell) and the Fc region of the antibody binds to an Fc receptor on an effector cell (e.g., a T effector cell). In mammals, humoral immune responses are mostly mediated by the interaction of the antibody Fc region with C1q and the complement cascade. Cellular immune responses are mostly mediated by the interaction of the antibody Fc region with the Fcγ receptor (i.e., FcγR). FcγRI, FcγRIIA, FcγRIIIA, and FcγRIIIB are activating FcγRs. FcγRIIB is inhibitory FcγR. Intracellular signaling of activating receptors is mediated by phosphorylation of intracellular ITAM motifs of the receptors, which leads to effector functions such as ADCC, ADCP, and inflammatory responses caused by inducing cytokine release. Intracellular signaling of the inhibitory receptor FcγRIIB is mediated by phosphorylation of the intracellular ITIM motif of the receptor, acting to balance the activating signaling pathway. The interaction of an antibody with FcγR and C1q is largely dependent on the hinge and the CH2 amino acid sequence and glycosylation of the CH2 region.

Therefore, in some embodiments, the antibody of the present invention comprises a heavy chain constant region that binds to activating FcγR. In a specific embodiment, the antibody comprises a heavy chain constant region that binds to an FcR selected from the following: FcγRIIIA (CD16a), an FcγRIIIA (CD16a) F158 variant, and an FcγRIIIA (CD16a) V158 variant. In another specific embodiment, the antibody comprises a constant region of the human IgG1 or IgG3 subclass. Preferably, the anti-TNFR2 antibody of the present invention induces ADCC in the presence of effector cells, such as NK cells.

In some preferred embodiments, the antibody of the present invention comprises an amino acid sequence of a human IgG1 constant region of SEQ ID NO: 62, or an amino acid sequence comprising at least one, two, or three but no more than 20, 10, or 5 amino acid changes relative to the amino acid sequence of SEQ ID NO: 62, or a sequence having at least 95%-99% identity to the amino acid sequence of SEQ ID NO: 62.

In a preferred embodiment, the antibody of the present invention comprises a light chain constant region. In a preferred embodiment, the light chain constant region is a human κ light chain constant region. In yet another preferred embodiment, the light chain constant region comprises an amino acid sequence of SEQ ID NO: 63, or an amino acid sequence having at least one, two, or three but no more than 20, 10, or 5 amino acid changes relative to the amino acid sequence of SEQ ID NO: 63, or an amino acid sequence having at least 95%-99% identity to the amino acid sequence of SEQ ID NO: 63.

In some preferred embodiments, the antibody of the present invention comprises a heavy chain comprising an amino acid sequence selected from SEQ ID NOs: 32-37 and 66, or an amino acid sequence having at least one, two, or three but no more than 20, 10, or 5 amino acid changes relative thereto, or an amino acid sequence having at least 80%, 85%, 90%, 92%, 95%, 97%, 98%, 99%, or more identity thereto. Preferably, the amino acid changes do not occur in the CDR regions, and more preferably, the amino acid changes do not occur in the variable regions.

In some preferred embodiments, the antibody of the present invention comprises a light chain comprising an amino acid sequence selected from SEQ ID NOs: 38-44 and 67, or an amino acid sequence having at least one, two, or three but no more than 20, 10, or 5 amino acid changes relative thereto, or an amino acid sequence having at least 80%, 85%, 90%, 92%, 95%, 97%, 98%, 99%, or more identity thereto. Preferably, the amino acid changes do not occur in the CDR regions, and more preferably, the amino acid changes do not occur in the variable regions.

In a preferred embodiment, the antibody of the present invention comprises a heavy chain sequence and/or a light chain sequence selected from:

(a) a heavy chain sequence comprising an amino acid sequence selected from SEQ ID NO: 32 or a variant thereof, and/or a light chain sequence comprising an amino acid sequence of SEQ ID NO: 38 or a variant thereof;

(b) a heavy chain sequence comprising an amino acid sequence selected from SEQ ID NO: 33 or a variant thereof, and/or a light chain sequence comprising an amino acid sequence of SEQ ID NO: 39 or 139 or a variant thereof;

(c) a heavy chain sequence comprising an amino acid sequence selected from SEQ ID NO: 34 or a variant thereof, and/or a light chain sequence comprising an amino acid sequence of SEQ ID NO: 40 or a variant thereof;

(d) a heavy chain sequence comprising an amino acid sequence selected from SEQ ID NO: 35 or a variant thereof, and/or a light chain sequence comprising an amino acid sequence of SEQ ID NO: 41 or a variant thereof;

(e) a heavy chain sequence comprising an amino acid sequence selected from SEQ ID NO: 36 or a variant thereof, and/or a light chain sequence comprising an amino acid sequence of SEQ ID NO: 42 or a variant thereof;

(f) a heavy chain sequence comprising an amino acid sequence selected from SEQ ID NO: 37 or a variant thereof, and/or a light chain sequence comprising an amino acid sequence of SEQ ID NO: 43 or 44 or a variant thereof,

(g) a heavy chain sequence comprising an amino acid sequence selected from SEQ ID NO: 66 or a variant thereof, and/or a light chain sequence comprising an amino acid sequence of SEQ ID NO: 67 or 44 or a variant thereof,

wherein the variant comprises an amino acid sequence comprising at least one, two or three but no more than 20, 10, or 5 amino acid changes, or having at least 80%, 85%, 90%, 92%, 95%, 97%, 98%, 99%, or more identity relative to the corresponding reference sequence. Preferably, the amino acid changes do not occur in the CDR regions, and more preferably, the amino acid changes do not occur in the variable regions.

In some embodiments, the heavy and/or light chains of the anti-TNFR2 antibody or the fragment thereof of the present invention further comprise a signal peptide sequence.

Exemplary antibody sequences

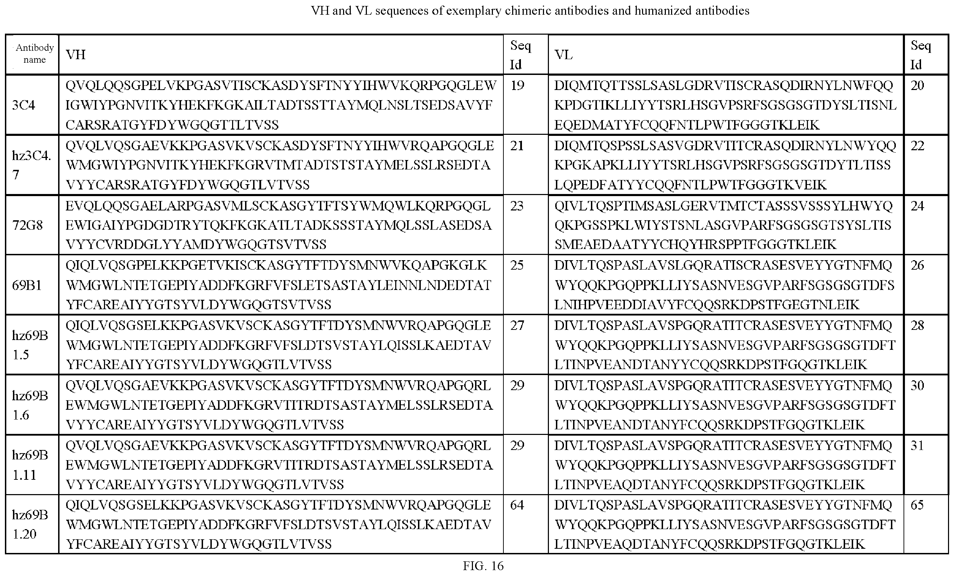

The present invention provides antibodies, e.g., murine, chimeric, and humanized antibodies, that specifically bind to TNFR2 (e.g., human TNFR2) as isolated and characterized in the examples. The antibody variable region VH and VL sequences of these exemplary antibodies of the present invention are listed in FIG 16. Exemplary CDR sequences of the antibodies are listed in FIG. 15.

Antibody variants

In one aspect, the present invention provides any of the antibodies described herein, particularly variants of the exemplary antibodies listed in Table B. In one embodiment, the antibody variant retains at least 60%, 70%, 80%, 90%, or 100% of the bioactivity (e.g., antigen-binding ability) of the antibody prior to change. In some embodiments, the change does not result in loss of the binding ability of an antibody variant to an antigen, and optionally may impart properties such as increased antigen affinity and different effector functions.

It can be understood that the heavy or light chain variable regions, or CDR regions of the antibody may be changed alone or in combination. In some embodiments, in terms of a target antibody sequence region, the antibody variant has at least 80%, 85%, 90%, 95%, or 99% or more amino acid identity to a reference antibody. For example, in one embodiment, the antibody of the present invention has at least 90%, 91%, 92%, 93%, 94%, 95%, 96%, 97%, 98%, or 99% or more identity relative to a reference antibody (e.g., one of the antibodies listed in Table 3) in terms of three heavy chain CDR regions. In one embodiment, the antibody of the present invention has at least 90%, 91%, 92%, 93%, 94%, 95%, 96%, 97%, 98%, or 99% or more identity relative to a reference antibody (e.g., one of the antibodies listed in Table 3) in terms of three light chain CDRs. In another embodiment, the antibody of the present invention has at least 90%, 91%, 92%, 93%, 94%, 95%, 96%, 97%, 98%, or 99% or more identity relative to a reference antibody (e.g., one of the antibodies listed in Table B) in terms of six CDR regions. In yet another embodiment, the antibody of the present invention has at least 80%, 85%, 90%, 91%, 92%, 93%, 94%, 95%, 96%, 97%, 98%, or 99% or more identity relative to a reference antibody (e.g., one of the antibodies listed in Table B) in terms of the heavy chain variable region. In yet another embodiment, the antibody of the present invention has at least 80%, 85%, 90%, 91%, 92%, 93%, 94%, 95%, 96%, 97%, 98%, or 99% or more identity relative to a reference antibody (e.g., one of the antibodies listed in Table B) in terms of the light chain variable region. In yet another embodiment, the antibody of the present invention has at least 80%, 85%, 90%, 91%, 92%, 93%, 94%, 95%, 96%, 97%, 98%, or 99% or more identity relative to a reference antibody (e.g., one of the antibodies listed in Table B) in terms of heavy and/or light chain variable regions.

In addition, changes may be made to an Fc region of the antibody. The changes to the Fc region may be made alone or in combination with the changes to the framework regions and/or CDRs described above. The Fc region can be changed, for example, to change one or more functions of the antibody, such as serum half-life, complement fixation, Fc receptor binding, and/or antigen-dependent cytotoxicity. In addition, the antibody of the present invention may be chemically modified (e.g., linked to PEG), or its glycosylation pattern may be changed.

In some other embodiments, depending on the desired antibody application, the antibody may comprise a modified Fc region variant having changed effector functions as compared to the unmodified parent Fc region. In general, the anti-TNFR2 antibody of the present invention having one or more changed (e.g., enhanced or reduced or eliminated) effector functions, such as an anti-TNFR2 antibody having an increased function selected from increased ADCC, CDC, ADCP, and/or serum half-life relative to an unmodified parent Fc region, can be obtained by linking the variable regions described herein to an Fc region comprising one or more modifications (amino acid substitutions, deletions, and/or insertions). In addition, the antibody can be chemically modified, for example, the glycosylation pattern of the antibody can be changed, to change its functional properties.

In certain embodiments, the antibody Fc region may comprise an Fc region having one or more amino acid replacements that improve the ADCC activity, e.g., replacements at positions 298, 333, and/or 334 (EU numbering of residues) of the Fc region. In some embodiments, the Fc region can also be changed to result in changed (i.e., increased or decreased) C1q binding and/or complement-dependent cytotoxicity (CDC) (see, e.g., US6,194,551, WO 99/51642 and Idusogie, E. E. et al., J. Immunol. 164 (2000) 4178-4184).

In some embodiments, the antibody comprises a variant Fc region having increased ADCC activity as compared to the native unmodified parent Fc region. Some examples of such Fc region variants comprise one or more amino acid modifications at a position selected from: 234, 235, 236, 238, 239, 240, 241, 243, 244, 245, 247, 248, 249, 252, 254, 255, 256, 258, 262, 263, 264, 265, 267, 268, 269, 270, 272, 276, 278, 280, 283, 285, 286, 289, 290, 292, 293, 294, 295, 296, 298, 299, 301, 303, 305, 307, 309, 312, 313, 315, 320, 322, 324, 325, 326, 327, 329, 330, 331, 332, 333, 334, 335, 337, 338, 340, 360, 373, 376, 378, 382, 388, 389, 398, 414, 416, 419, 430, 433, 434, 435, 436, 437, 438, or 439 (amino acid residues numbered according to EU index). Exemplary alternatives include: 236A, 239D, 239E, 268D, 267E, 268E, 268F, 324T, 332D, and 332E. Exemplary combinatorial alternatives include 239D/332E, 236A/332E, 236A/239D/332E, 268F/324T, 267E/268F, 267E/324T, and 267E/268F/324T.

Binding sites on human IgG1 to FcγR1, FcγRII, FcγRIII, and FcRn have been mapped and variants with improved binding have been described (see Shields, R. L. et al. (2001) J. Biol. Chem. 276: 6591-6604). Specific mutations at positions 256, 290, 298, 333, 334, and 339 are shown to improve binding to FcyRIII. The following combinatorial mutants are shown to improve FcyRIII binding and ADCC activity: T256A/S298A, S298A/E333A, S298A/K224A, S298A/E333A/K334A, S239D/I332E, and S239D/I332E/A330L.

In some other embodiments, the Fc region can be changed to increase or decrease its glycosylation degree and/or change its glycosylation pattern. Addition or deletion of glycosylation sites of the Fc region can be conveniently achieved by producing or removing one or more glycosylation sites through amino acid sequence changes. For example, one or more amino acid substitutions may be made to eliminate one or more glycosylation sites, thereby eliminating the glycosylation at the sites. Antibodies with changed types of glycosylation can be prepared, such as low-fucosylated or non-fucosylated antibodies with reduced amount of fucosyl residues or antibodies with increased bisecting GlcNac structures. Such changed glycosylation patterns have shown the ability to increase ADCC of antibodies. Herein, antibody variants having at least one galactose residue in the oligosaccharide linked to the Fc region are also considered. The antibody variants may have an increased CDC function.

Additionally or alternatively, change of the glycosylation degree and/or pattern of the antibody can be achieved, for example, by expressing the antibody in a host cell with a changed glycosylation mechanism. Cells with the changed glycosylation mechanism have been described in the art and can be used as host cells in which recombinant antibodies are expressed to produce antibodies with changed glycosylation.

In some other embodiments, the present invention also considers antibody variants having some but not all effector functions, which makes them a desirable candidate for some applications in which the half-life period of the antibody *in vivo* is important while certain effector functions (such as complement and ADCC) are unnecessary or deleterious. For example, the Fc region may comprise a mutation that eliminates or reduces effector functions, such as the human IgG1 Fc region with mutations P329G and/or L234A and L235A, or the human IgG4 Fc region with mutations P329G and/or S228P and L235E.

In certain embodiments, antibodies modified by cysteine engineering may need to be produced, such as "sulfo-MAb", wherein one or more residues of the antibodies are replaced by cysteine residues. For example, the number of cysteine residues in the hinge region of an antibody can be changed, e.g., to facilitate the assembly of the light and heavy chains or to increase or decrease the stability of the antibody. See, e.g., U.S. Pat. No. 5,677,425 for residues.

In certain embodiments, the antibodies provided herein can be further modified to comprise other non-protein portions. Suitable portions for antibody derivatization include, but are not limited to, water-soluble polymers. Non-limiting examples of water-soluble polymers include, but are not limited to, polyethylene glycol (PEG) to, e.g., increase the (e.g., serum) half-life of an antibody. Methods for protein PEGylation are known in the art and can be applied to the antibodies of the present invention. See, e.g., EP 0154316 and EP 0401384.

## II. **Polynucleotide, Vector, and Host**

The present invention provides a nucleic acid encoding any of the anti-TNFR2 antibodies or the fragments thereof, and also provides a vector comprising the nucleic acid. In one embodiment, the vector is an expression vector. In addition, a host cell comprising the nucleic acid or the vector is provided. In one embodiment, the host cell is eukaryotic. In another embodiment, the host cell is selected from a yeast cell and a mammalian cell (e.g., a CHO cell or a 293 cell). In another embodiment, the host cell is prokaryotic.

In one aspect, the present invention provides a nucleic acid encoding any of the anti-TNFR2 antibodies or the fragments thereof. The nucleic acid can include a nucleic acid encoding an amino acid sequence of the light chain variable region and/or heavy chain variable region of the antibody, or a nucleic acid encoding an amino acid sequence of the light chain and/or heavy chain of the antibody. An exemplary nucleic acid sequence encoding the heavy chain variable region of the antibody comprises a nucleic acid sequence having at least 80%, 85%, 90%, 91%, 92%, 93%, 94%, 95%, 96%, 97%, 98%, or 99% identity to a nucleic acid sequence selected from SEQ ID NOs: 45, 47, 49, 51, 53, 55, and 68, or a nucleic acid sequence selected from SEQ ID NOs: 45, 47, 49, 51, 53, 55, and 68. An exemplary nucleic acid sequence encoding the light chain variable region of the antibody comprises a nucleic acid sequence having at least 80%, 85%, 90%, 91%, 92%, 93%, 94%, 95%, 96%, 97%, 98%, or 99% identity to a nucleic acid sequence selected from SEQ ID NOs: 46, 48, 50, 52, 54, 56, 57, and 69, or a nucleic acid sequence selected from SEQ ID NOs: 46, 48, 50, 52, 54, 56, 57, and 69. Polypeptides encoded by the polynucleotides can show antigen-binding (TNFR2-binding) ability when expressed in a suitable expression vector.

The present invention also provides a polynucleotide encoding at least one CDR region and typically all three CDR regions from a heavy chain (VH) sequence or a light chain (VL) sequence of the antibodies binding to TNFR2 described above. In some further embodiments, the polynucleotide encodes the complete or substantially complete variable region sequence of the heavy chain and/or the light chain of the antibody that binds to TNFR2 described above.

As will be understood by those skilled in the art, each antibody or polypeptide amino acid sequence can be encoded by a variety of nucleic acid sequences because of codon degeneracy.

In a preferred embodiment, the nucleic acid encoding the antibodies of the present invention further comprises a nucleotide sequence encoding a heavy chain constant region, e.g., a constant region sequence set forth in SEQ ID NO: 62 or a sequence substantially identical thereto.

In a preferred embodiment, the nucleic acid encoding the antibodies of the present invention further comprises a nucleotide sequence encoding a light chain constant region, e.g., a sequence set forth in SEQ ID NO: 63 or a sequence substantially identical thereto.

The above nucleotide sequences for the antibody can be generated by *de novo* solid phase DNA synthesis or by PCR mutagenesis of sequences encoding the antibody or the antigen-binding fragment thereof that binds to TNFR2, using methods well known in the art.

In one embodiment, one or more vectors comprising the nucleic acid of the present invention are provided. In one embodiment, the vector is an expression vector, e.g., a eukaryotic expression vector. The vector includes, but is not limited to, a virus, a plasmid, a cosmid, a λ phage, or a yeast artificial chromosome (YAC).

In one embodiment, provided is a host cell comprising the vector. The suitable host cell for cloning or expressing the vector encoding the antibody includes prokaryotic cells or eukaryotic cells described herein. For example, the antibody may be produced in bacteria, particularly when glycosylation and Fc effector functions are not required. Expression of antibody fragments and polypeptides in bacteria is described in, for example, U.S. Pat. Nos. 5648237, 5789199, and 5840523, and also described in Charlton, Methods in Molecular Biology, Vol. 248 (B. K. C. Lo, eds., Humana Press, Totowa, NJ, 2003), pg. 245-254, which describes the expression of antibody fragments in *E. coli*. After expression, the antibody can be isolated from bacterial cell paste in soluble fraction and can be further purified.

In one embodiment, the host cell is eukaryotic. In another embodiment, the host cell is selected from a yeast cell, a mammalian cell, or other cells suitable for preparing an antibody or an antigen-binding fragment thereof. For example, eukaryotic microorganisms, such as filamentous fungi or yeast, are suitable cloning or expression hosts for the vector encoding the antibody. For example, fungus and yeast strains in which a glycosylation pathway has been "humanized" may produce antibodies having a partial or full human glycosylation pattern. See Gerngross, Nat. Biotech., 22: 1409-1414 (2004), and Li et al., Nat. Biotech., 24: 210-215 (2006). Host cells suitable for expressing a glycosylated antibody are also derived from multicellular organisms (invertebrates and vertebrates). Vertebrate cells may also be used as hosts. For example, a mammalian cell line engineered to be suitable for suspension growth may be used. Other examples of useful mammalian host cell lines are monkey kidney CV1 lines (COS-7) transformed with SV40, human embryonic kidney lines (293HEK or 293 cells, as described in, e.g., Graham et al., J. Gen Virol. 36: 59 (1977)) and the like. Other useful mammalian host cell lines include Chinese hamster ovary (CHO) cells, including DHFR-CHO cells (Urlaub et al., Proc. Natl. Acad. Sci. USA 77: 216 (1980)), and myeloma cell lines such as Y0, NS0, and Sp2/0. For reviews of certain mammalian host cell lines suitable for antibody production, see, e.g., Yazaki and Wu, Methods in Molecular Biology, Vol. 248 (B. K. C. Lo, ed., Humana Press, Totowa, NJ), p. 255-268 (2003).

## III. Preparation of antibodies

In one embodiment, provided is a method for preparing the anti-TNFR2 antibody, comprising culturing a host cell comprising a nucleic acid encoding an antibody under a condition suitable for expressing the antibody, as provided above, and optionally recovering the antibody from the host cell (or a host cell medium). For recombinant production of the anti-TNFR2 antibody, a nucleic acid encoding the antibody (e.g., the antibody described above) is isolated and inserted into one or more vectors for further cloning and/or expression in the host cells. Such nucleic acids can be easily isolated and sequenced by using conventional procedures (e.g., by using oligonucleotide probes that are capable of specifically binding to genes encoding heavy and light chains of antibodies).

## IV. Assay

The anti-TNFR2 antibody provided herein can be identified, screened, or characterized for physical/chemical properties and/or bioactivity thereof through a variety of assays known in the art.

For example, the binding of an antibody to human TNFR2 can be determined by methods known in the art, such as ELISA, Western blot, and the like, or by the exemplary methods disclosed in the examples herein. For example, the assay can be performed by flow cytometry, wherein the antibody reacts with a cell line expressing human TNFR2, e.g., CHO cells transfected to express human TNFR2 on the cell surface. Other cells are also suitable for flow cytometry, including tumor cells or primary Treg cells expressing human TNFR2. Alternatively, the binding of the antibody, including binding kinetics (e.g., $K_D$), can be determined in a biological optical interferometry assay using a recombinant TNFR2 protein. In some embodiments, the equilibrium dissociation constant for the binding of the antibody to TNFR2 from human and other species is determined by biological optical interferometry (e.g., Fortebio affinity measurement).

The inhibitory/antagonistic antibodies against TNFR2 of the present invention can block the TNFR2 signaling pathway activated by a ligand TNF$\alpha$ by binding to TNFR2 on the cell membrane. In some embodiments, the blocking activity is dependent on the blocking of the binding of TNFR2 to its natural ligand TNF$\alpha$ by the antibody, for example, the example antibodies 3C4 and 69B1 and humanized forms thereof, or antibodies having the same or similar VH and VL sequences thereto. In some other embodiments, the blocking activity is independent of the blocking of the binding of TNFR2 to its natural ligand TNF$\alpha$ by the antibody, for example, the antibody 72G8 and humanized forms thereof, or antibodies having the same or similar VH and VL sequences thereto, which can directly inhibit polymerization of TNFR2 on the cell surface, thereby inhibiting the activation of a TNFR2 receptor. The inhibitory effect of the antibodies of the present invention on the TNF$\alpha$-activated TNFR2 signaling pathway can be determined in Jurkat cells overexpressing TNFR2 by, for example, the Jurkat TNFR2 NF-$\kappa$B luciferase reporter system. In some embodiments, the inhibitory effect of the antibodies of the present invention on the TNF$\alpha$-activated TNFR2 signaling pathway is determined according to the method described in Example 8.

The ADCC-killing effect of the antibodies of the present invention on target cells expressing TNFR2 can be determined by the ADCC reporter bioactivity assay. In the ADCC reporter bioactivity assay, earlier events during activation of the pathway by the ADCC mechanism are selected as readout indicators of the assay: i.e. the activation of gene transcription mediated by the NFAT (nuclear factor of activated T cells) pathway in effector cells. In addition, in the ADCC reporter assay, engineered T cells were used as effector cells (e.g., Jurkat cells) that stably express an FcyRIIIa receptor (e.g., V158 high-affinity mutant) and firefly luciferase expressed by driving by NFAT response elements. In the assay, the bioactivity of the antibodies in the ADCC mechanism is quantified by luciferase produced by the activation of the NFAT pathway, whereas luciferase activity in effector cells is quantified by bioluminescence readings. In the ADCC reporter bioactivity assay, a good assay response is obtained only if target cells with the correct surface antigen, the correct specific antibody, and effector cells expressing FcγRIIIa are present together. In a preferred embodiment, the ADCC activity of the antibodies of the present invention is determined by the ADCC reporter assay described in Example 14.

The present invention also provides an assay for identifying anti-TNFR2 antibodies having bioactivities. The biological activities can include, for example, binding to TNFR2 (e.g., binding to human TNFR2), blocking the binding of TNFR2 (e.g., binding of human TNFR2) to a TNF$\alpha$ molecule, inhibiting signaling mediated by the binding of TNF$\alpha$ to TNFR2, eliciting ADCC activity, and/or inhibiting tumor growth. For example, the ability of antibodies in inhibiting tumor growth is tested in an *in vivo* tumor inhibition model (see, e.g., Examples 15 and 16). Antibodies having such bioactivity *in vivo* and/or *in vitro* are also provided herein.

It can be understood that any of the above assays can be performed using the immunoconjugates or multispecific antibodies of the present invention to replace or supplement anti-TNFR2 antibodies.

### V. Multispecific Antibodies

In yet another aspect, the present invention provides a multispecific (including bispecific) antibody molecule that specifically binds to TNFR2 (preferably human TNFR2). In one embodiment, in the multispecific antibody, the antibody (or the antigen-binding fragment thereof) of the present invention has a first binding specificity for TNFR2. In yet another embodiment, the multispecific antibody further comprises a second specificity for one of the following molecules, or further comprises second and third binding specificities for both of the following molecules. The second and third binding specificities may, for example, be directed to another antigen expressed on the surface of a tumor cell.

In one embodiment, the binding specificity is dependent on a "binding site" or an "antigen-binding site" (a region of an antibody molecule that actually binds to an antigen) of the antibodies. In a preferred embodiment, the antigen-binding site is formed by a VH/VL pair consisting of a light chain variable domain (VL) and a heavy chain variable domain (VH) of the antibodies. Thus, in one embodiment, the "multispecific" antibodies have at least two antigen-binding sites, each of which can bind to a different epitope of the same antigen or to a different epitope of a different antigen. For multispecific antibodies and preparation thereof, reference may be made to, for example, the descriptions in WO 2009/080251, WO 2009/080252, WO 2009/080253, and WO 2010/145793.

### VI. Immunoconjugate

In yet another aspect, the present invention provides an immunoconjugate produced by conjugating the antibody of the present invention to a heterologous molecule. In one embodiment, in the immunoconjugate, the antibody (or the antigen-binding fragment thereof) of the present invention is conjugated to a therapeutic or diagnostic agent. In some embodiments, the antibody of the present invention can be conjugated in the form of a full-length antibody or an antibody fragment to a heterologous molecule. For example, the antibody is conjugated in the form of a Fab fragment, a Fab' fragment, a F(ab)'$_2$ fragment, a single-chain scFab antibody, a single-chain scFv, or other fragments.

A linker can be used to covalently link different entities of the conjugate. Suitable linkers include chemical linkers or peptide linkers. Advantageously, the linker is a "cleavable linker" that facilitates the release of the polypeptides following delivery to a target site. For example, acid-labile linkers, peptidase-sensitive linkers, photolabile linkers, dimethyl linkers, or disulfide-comprising linkers can be used (Chari et al., Cancer Research, 52 (1992) 127-131; US 5,208,020).

Therapeutic agents suitable for use in the conjugate include, but are not limited to, cytotoxins (e.g., cytostatic agents or cell killers), drugs, or radioisotopes. Examples of the cytotoxic agent (such as a chemotherapeutic agent) suitable for forming the immunoconjugate are known in the art, see, e.g., WO 05/103081. For example, the cytotoxic agent includes, but is not limited to: radioisotopes; growth inhibitors; enzymes and fragments thereof such as nucleases; antibiotics; toxins such as small-molecule toxins or enzymatically active toxins of bacterial, fungal, plant, or animal sources, including fragments and/or variants thereof; and a variety of known anti-tumor or anti-cancer agents.

In yet another aspect, the antibody of the present invention can be conjugated to a diagnostic or detectable agent. Such conjugates can be used as part of a clinical testing method (e.g., to determine the efficacy of a particular therapy) to monitor or predict the onset, formation, progression, and/or severity of a disease or disorder. Such diagnosis and detection can be achieved by coupling the antibody to the detectable agent, which includes, but is not limited to, a variety of enzymes, such as but not limited to, horseradish peroxidase; prosthetic groups, such as but not limited to, streptavidin/biotin and avidin/biotin; fluorescent substances; luminescent substances; radioactive substances; and positron-emitting metals and non-radioactive paramagnetic metal ions used in various positron emission tomography techniques.

**VII. Pharmaceutical Composition and Pharmaceutical Preparation**

The present invention further comprises a composition (including a pharmaceutical composition or a pharmaceutical preparation) comprising the anti-TNFR2 antibody or the immunoconjugate thereof or the multispecific antibody, and a composition comprising a polynucleotide encoding the anti-TNFR2 antibody or the immunoconjugate thereof or the multispecific antibody. Such compositions can further optionally comprise suitable pharmaceutical auxiliary materials, such as a pharmaceutical carrier and a pharmaceutical excipient known in the art, including buffers.

The pharmaceutical carrier suitable for use in the present invention may be sterile liquid, such as water and oil, including those derived from petroleum, animals, plants, or synthesis, such as peanut oil, soybean oil, mineral oil, and sesame oil. Water is a preferred carrier when the pharmaceutical composition is administered intravenously. Saline solutions, aqueous dextrose, and glycerol solutions can also be used as liquid carriers, particularly for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol, etc. For use and application of excipients, see also Handbook of Pharmaceutical Excipients, 5th edition., R. C. Rowe, P. J. Seskey and S. C. Owen, Pharmaceutical Press, London, Chicago. The composition may further comprise a small amount of wetting agent, emulsifier, or pH buffer, if desired. The compositions may be in the form of a solution, a suspension, an emulsion, a tablet, a pill, a capsule, a powder, a sustained release preparation, or the like. Oral preparations may comprise standard carriers, such as pharmaceutical grade mannitol, lactose, starch, magnesium stearate, and saccharin.

A pharmaceutical preparation comprising the present invention can be prepared by mixing the anti-TNFR2 antibody immunoconjugate, or multispecific antibody of the present invention of a desired purity with one or more optional pharmaceutical auxiliary materials, preferably in the form of a lyophilized preparation or an aqueous solution (Remington's Pharmaceutical Sciences, 16th edition, Osol, A. eds. (1980)).

In the pharmaceutical composition and pharmaceutical preparation of the present invention, the antibody of the present invention may be the sole active agent, or may be combined with additional therapeutic agents. Therapeutic agents that may be combined with the antibody of the present invention include, but are not limited to, therapeutic agents that have beneficial therapeutic effects for the disease and/or disorder to be treated. For example, the active ingredients may be those required for a particular indication being treated, preferably those having complementary activities that do not adversely affect each other. For example, other pharmaceutical ingredients that provide anti-cancer activity may be provided. The antibody of the present invention is suitably present in combination with an active ingredient in an amount effective for the intended use in the pharmaceutical composition and the pharmaceutical preparation.

A sustained release preparation can be prepared. Suitable examples of the sustained release preparation include a semipermeable matrix of a solid hydrophobic polymer containing an antibody. The matrix is in the form of a shaped article, such as a film or a microcapsule.

For other components of the pharmaceutical preparation, see also those disclosed in WO 2015/153513.

**VIII. Combination Product**

In yet another aspect, the present invention also provides a combination product comprising the antibody or the antigen-binding fragment thereof, the bispecific antibody, or the immunoconjugate of the present invention, and one or more additional therapeutic agents (e.g., a chemotherapeutic agent, other antibodies, a cytotoxic agent, an anti-tumor drug). The combination product of the present invention can be used in the treatment method of the present invention. In some embodiments, the present invention provides a combination product, wherein the additional therapeutic agent is, e.g., a therapeutic agent, such as an antibody, which is effective to stimulate an immune response and thus further enhances, stimulates, or upregulates the immune response in a subject.

In some embodiments, the combination product is used for preventing or treating a tumor. In some embodiments, the tumor is a cancer, e.g., colon cancer or chronic myeloid leukemia.

**IX. Method and Use**

In one aspect, the present invention provides a method and use of the TNFR2 antibody or the antigen-binding fragment thereof of the present invention, e.g., *in vivo* and *in vitro,* for:

(1) blocking the binding of TNFR-2 to its ligand TNFα;
(2) directly inhibiting polymerization of TNFR2 without blocking the binding of TNFR-2 to its ligand TNFα;
(3) inhibiting activation of a TNFR2 receptor in a manner dependent on or independent of blocking of the binding of TNFα to TNFR2;
(4) inhibiting activation of NF-xB signaling pathway mediated by the binding of TNFα to TNFR2;

(5) antagonizing a Treg cell proliferation promoting effect of a natural agonist TNFα of TNFR2; and
(6) depending on ADCC activity elicited by an antibody Fc region, killing tumor cells expressing TNFR2 on the surface.

In some embodiments, the method and use of the present invention relate to the treatment of a disease in an individual subject. In some other embodiments, the method and use of the present invention relate to the detection of the presence of TNFR2 in a sample from, for example, a subject. In still other embodiments, the present invention also provides use of the TNFR2 antibody or the antigen-binding fragment thereof of the present invention in preparing a product (e.g., a pharmaceutical composition or a pharmaceutical product or a detection product) for the purposes described above.

Therapeutic applications

The present invention provides a method and use of the antibody or the antigen-binding fragment thereof of the present invention for preventing or treating a TNFR2-associated disease, including but not limited to, a tumor, such as colon cancer or chronic myeloid leukemia, in an individual or subject. In some embodiments, the antibody of the present invention may be administered as the sole active agent or may be administered in combination with other therapies or therapeutic agents. Such other therapies and therapeutic agents include, for example, drugs that target antigens on the surface of tumor cells to destroy tumors by binding to and/or blocking these molecules; drugs that activate the immune system of a subject to enable the immune system to spontaneously destroy tumors.

As used herein, the terms "individual" and "subject" are used interchangeably and refer to a mammal. The mammals include, but are not limited to, domesticated animals (e.g., cows, sheep, cats, dogs, and horses), primates (e.g., human and non-human primates such as monkeys), rabbits, and rodents (e.g., mice and rats). In particular, a subject is a human.

As used herein, "treatment" includes 1) therapeutic measures, which cure, slow, alleviate symptoms of, and/or stop the progression of the diagnosed pathological condition or disorder; and 2) prophylactic or preventative measures, which prevent and/or slow the progression of the pathological condition or disorder. Therefore, subjects in need of treatment include a subject who already has the disorder, a subject who is susceptible to the disorder, and a subject who is intended to prevent the disorder. The subject will benefit from the therapeutic or prophylactic measures and exhibit a reduction or amelioration in the development, recurrence, or progression of a disease, disorder, condition, and/or symptom as compared to a subject who does not receive the treatment. In some embodiments, the present invention relates to treatment of a disease or disorder; in some other embodiments, the present invention relates to prevention of a disease or disorder.

In some embodiments according to the present invention, "treatment" of a disease or disorder refers to ameliorating the disease or disorder (i.e., slowing or arresting or reducing the progression of the disease or at least one clinical symptom thereof). In some other embodiments, "treatment" refers to ameliorating or improving at least one physical parameter, including those physiological parameters that may not be discernible by the patient. In some other embodiments, "treatment" refers to modulating the disease or disorder, either physically (e.g., stabilization of a discernible symptom), physiologically (e.g., stabilization of a physical parameter), or both. Unless explicitly described herein, methods for assessing treatment and/or prevention of a disease or disorder are generally known in the art.

In still other embodiments according to the present invention, "treatment" of a disease or disorder refers to "prevention" of the disease or disorder, including inhibition of the development or progression of the disease or disorder or a symptom of a particular disease or disorder. In some embodiments, the subject is a candidate for a prophylactic regimen. In general, the term "prevention" refers to the administration of a drug prior to the development of a sign or symptom of a disease or disorder, particularly in subjects at risk of the disease.

The terms "therapeutically effective amount", "prophylactically effective amount", or "effective amount" herein refer to an amount of the anti-TNFR2 antibody or the antigen-binding fragment thereof of the present invention that is effective to prevent or ameliorate the development of one or more symptoms of a disease or condition, or the disease or condition, when administered to a cell, tissue, or subject, alone or in combination with additional therapeutic agents. The therapeutically effective amount also refers to an amount of the antibody or the antigen-binding fragment thereof sufficient to cause amelioration of symptoms, e.g., an amount for treating, curing, preventing, or ameliorating a related condition or promoting the treatment, cure, prevention, or amelioration of such condition. When an active ingredient is to an individual alone, a therapeutically effective amount only refers to the amount of the ingredient. When more than one active ingredient is administered in combination, a therapeutically effective amount refers to the combined amount of the active ingredients that results in the therapeutic effect, whether administered concurrently, sequentially, or simultaneously. An effective amount of the therapeutic agent will result in an increase in a diagnostic standard or parameter by at least 10%, typically at least 20%, preferably at least about 30%, more preferably at least 40%, and most preferably at least 50%.

Therefore, in some embodiments, the present invention relates to a method for inhibiting a tumor in a subject, comprising administering to the subject an effective amount of any of the anti-TNFR2 antibodies or the fragments thereof described herein, an immunoconjugate or a multispecific antibody comprising the antibodies or the fragments thereof, or a pharmaceutical composition. In some other embodiments, the present invention also relates to a method for enhancing an anti-tumor immune response of the body of a subject, comprising administering to the subject an effective amount of any of the anti-TNFR2 antibodies or the fragments thereof described herein, an immunoconjugate or a multispecific antibody comprising the antibodies or the fragments thereof, or a pharmaceutical composition. In some embodiments, the anti-TNFR2 antibody or the antigen-binding moiety thereof of the present invention is administered to a tumor-bearing subject for killing the tumor-bearing TNFR2. In some embodiments, the anti-TNFR2 antibody or the antigen-binding fragment thereof of the present invention is administered to a subject bearing a tumor to stimulate an anti-tumor immune response.

In some other embodiments, the present invention provides a method for treating a tumor, e.g., a cancer, in a subject, comprising administering to the subject an effective amount of any of the anti-TNFR2 antibodies or the fragments thereof described herein, an immunoconjugate or a multispecific antibody comprising the antibodies or the fragments thereof, or a pharmaceutical composition. The cancer may be at an early, intermediate, or advanced stage, or may be a metastatic cancer.

Tumors that may be treated by the methods of the present invention include, but are not limited to, e.g., colon cancer and chronic myeloid leukemia. The antibody can inhibit the growth of a tumor by targeting tumor cells and/or Treg cells expressing TNFR2 on the cell surface.

The antibody of the present invention (the pharmaceutical composition or the immunoconjugate comprising the same, and any additional therapeutic agent) can be administered by any suitable method, including parenteral administration, intrapulmonary administration, intranasal administration, and, if required by locoregional treatment, intralesional administration. Parenteral infusion includes intramuscular, intravenous, intra-arterial, intraperitoneal, or subcutaneous administration. The administration may be performed by any suitable route, such as injection, e.g., intravenous or subcutaneous injection, to some extent depending on short-term or long-term treatment. Various administration schedules are encompassed herein, including, but not limited to, single administration or multiple administrations at multiple time points, bolus injection, and pulse infusion.

In order to prevent or treat diseases, the appropriate dosage of the antibody of the present invention (when used alone or in combination with one or more additional therapeutic agents) will depend on types of diseases to be treated, types of antibodies, severity, and progression of the disease, purpose of administration (prophylactic or therapeutic) of the antibody, previous treatments, clinical histories of patients, responses to the antibody, and the discretion of an attending physician. The antibody is suitably administered to a patient through a single treatment or through a series of treatments.

In the methods described above, the composition, multispecific antibody, or immunoconjugate of the present invention can be administered in place of the antibody or the antigen-binding fragment thereof of the present invention. Alternatively, in the methods, the composition, multispecific antibody, or immunoconjugate of the present invention can be further administered after the antibody or the antigen-binding fragment thereof of the present invention is administered.

In some embodiments, the present invention provides use of the anti-TNFR2 antibody, the composition, the immunoconjugate, and the multispecific antibody of the present invention preparing a drug used for the methods described above (e.g., used for treatment).

Detection application

The present invention also provides a method and a kit for detecting TNFR2 in a sample, comprising: (a) contacting the sample with the antibody or the antigen-binding fragment thereof or the immunoconjugate of the present invention; and (b) detecting a complex formed by the antibody or the antigen-binding fragment thereof or the immunoconjugate with a TNFR2 protein. In some embodiments, the sample is from a cancer patient, e.g., a skin cancer patient. The detection may be *in vitro* or *in vivo.*

The term "detection" used herein includes quantitative and qualitative detections, and exemplary detections may involve immunohistochemistry, immunocytochemistry, flow cytometry (e.g., FACS), magnetic beads complexed with antibody molecules, ELISA, and PCR (e.g., RT-PCR). In certain embodiments, the biological sample is blood, serum, or other liquid samples of biological origin. In certain embodiments, the biological sample includes cells or tissues. In some embodiments, the biological sample is derived from a hyperproliferative or cancerous lesion. In certain embodiments, the TNFR2 to be detected is human TNFR2.

In one embodiment, the anti-TNFR2 antibody is used to select a subject suitable for treatment with the anti-TNFR2 antibody, e.g., wherein TNFR2 is a biomarker for selecting the subject. In one embodiment, the antibody of the present invention can be used to diagnose a cancer or tumor, e.g., to assess (e.g., monitor) the treatment or progression, diagnosis, and/or staging of a disease (e.g., the hyperproliferative or cancerous disease) described herein in a subject.

In certain embodiments, a labeled anti-TNFR2 antibody is provided. The label includes, but is not limited to, a label or moiety that is detected directly, e.g., a fluorescent label, a chromophoric label, an electron-dense label, a chemiluminescent label, and a radioactive label, and a moiety that is detected indirectly, such as an enzyme or a ligand, for example, by enzymatic reaction or molecular interaction. Exemplary labels include, but are not limited to, radioisotopes $^{32}$P, $^{14}$C, $^{125}$I, $^{3}$H, and $^{131}$I, fluorophores (such as rare earth chelates or fluorescein) and derivatives thereof, rhodamine and derivatives thereof, dansyl, umbelliferone, luceriferase (such as firefly luciferase and bacterial luciferase (U.S. Patent No. 4,737,456)), fluorescein, 2,3-dihydrophthalazinedione, horseradish peroxidase (HR), alkaline phosphatase, β-galactosidase, glucoamylase, lysozyme, carbohydrate oxidase (such as glucose oxidase, galactose oxidase, and glucose-6-phosphate dehydrogenase), heterocyclic oxidase (such as uricase and xanthine oxidase), enzymes oxidizing dye precursors with hydrogen peroxide (such as HR, lactoperoxidase, or microperoxidase), biotin/avidin, spin labels, phage labels, stable free radicals, etc.

The following examples are described to assist in understanding the present invention. The examples are not intended to be and should not be interpreted in any way as limiting the protection scope of the present invention.

Examples

**Example 1. Preparation of Hybridoma Cells**

In the experiment, mice were immunized by a human TNFR2 protein, and then spleen cells of the mice were obtained and fused with myeloma cells to obtain hybridoma cells capable of expressing positive antibodies.

Hybridoma fusion

Experimental animal and immunization method

| | |
| --- | --- |
| Mice | Balb/c (Beijing Vital River Laboratory Animal Technology Co., Ltd.) |
| Immunization antigen | Human TNFR2 protein, Sino biological, Catalog No. 10417-H03H |
| Immunization method | 50 μg/mouse, subcutaneous injection (SC), 50 μL per spot, 2 spots |
| Number of immunizations | 5 |

Cell fusion:
Spleens were taken from the immunized animals to prepare a spleen cell suspension. After filtration of the spleen cell suspension and lysis of red blood cells, spleen cells were suspended in 20 mL of basal medium and counted.

| Name | Composition | Preparation |
| --- | --- | --- |
| Basal medium | FBS (Hyclone,SH30084.03) | 10% |
| | GlutaMAX™ Supplement (Gibco, 35050-079) | 1× |
| | RPMI-1640 (Hyclone, SH30809.01) | 90% |

Mouse myeloma cells SP2/0 (ATCC, CRL-1581) were resuspended in 20 mL of basal medium and counted. The SP2/0 cells and spleen cells were mixed in a ratio of 1:2 to 1:1, and the mixture was centrifuged at 1000 rpm for 6 min. After the supernatant was removed, the mixed cells were resuspended in 10 mL of fusion buffer (BTXpress, 47-0001). Then, 15 mL of fusion buffer was added, the mixture was centrifuged at 1000 rpm for 5 min, and the supernatant was removed. The above steps were repeated once. The cells were resuspended in an appropriate amount of fusion buffer, and the density of mixed cells was adjusted to $1 \times 10^7$ cells/mL. The settings of the electrofusion apparatus were as follows. 2 mL of cell suspension was added to each dish for electrofusion.

| Condition | Mouse (SP2/0-ECF-F) |
| --- | --- |
| Alignment: | 60v, 30 sec |
| Membrane breaking: | 1500V, 30μs, 3X |
| Post-fusion pulse: | 60V, 3 sec |

Screening after electrofusion:

The cells were left to stand in the dish at room temperature for 5 min. The cells were transferred to a centrifuge tube and diluted to 1-2×10⁴ cells/mL with a screening medium (prepared according to the table below). 100 μL of cell suspension was added to each well of a 96-well plate. The screening medium was changed 7 days after the fusion. Cells were screened on Day 10 (or later, depending on the cell growth state) of the culture. Hybridoma cells expressing specific anti-hTNFR2 antibodies were screened by ELISA.

| Name | Composition | Preparation |
|---|---|---|
| Screening medium | RPMI-1640 (Hyclone, SH30809.01) | 80% |
| | FBS(Hyclone,SH30084.03) | 20% |
| | HAT medium (Gibco,21060-017) | 1× |
| | GlutaMAX™ Supplement (Gibco Gibco, 35050-079) | 1× |

The antibodies expressed by the selected hybridoma cells were sequenced by PCR to determine the variable region genes of the antibodies, and chimeric antibody screening, humanization and affinity maturation were performed to obtain candidate sequences at different stages.

The amino acid sequences of CDR regions, light and heavy chain variable regions, and light and heavy chains of 3 exemplary antibodies (3C4, 69B1, and 72G8) of the present invention and humanized forms thereof, as well as corresponding nucleotide sequences, are listed in the table below.

Table 1. SEQ ID NOs of related sequences of the antibodies involved in the present invention

| Antibody name | HCDR1 | HCDR2 | HCDR3 | LCDR1 | LCDR2 | LCDR3 | VH | VL | HC | LC | VH DNA | VL DNA |
|---|---|---|---|---|---|---|---|---|---|---|---|---|
| 3C4 | 1 | 2 | 3 | 4 | 5 | 6 | 19 | 20 | 32 | 38 | 45 | 46 |
| hz3C4.7 | 1 | 2 | 3 | 4 | 5 | 6 | 21 | 22 | 33 | 39 | 47 | 48 |
| 72G8 | 7 | 8 | 9 | 10 | 11 | 12 | 23 | 24 | 34 | 40 | 49 | 50 |
| 69B1 | 13 | 14 | 15 | 16 | 17 | 18 | 25 | 26 | 35 | 41 | 51 | 52 |
| hz69B1.5 | 13 | 14 | 15 | 16 | 17 | 18 | 27 | 28 | 36 | 42 | 53 | 54 |
| hz69B1.6 | 13 | 14 | 15 | 16 | 17 | 18 | 29 | 30 | 37 | 43 | 55 | 56 |
| hz69B1.11 | 13 | 14 | 15 | 16 | 17 | 18 | 29 | 31 | 37 | 44 | 55 | 57 |
| hz69B1.20 | 13 | 14 | 15 | 16 | 17 | 18 | 64 | 65 | 66 | 67 | 68 | 69 |
| Note: HC was formed by fusing a VH sequence to an IgG1 portion of SEQ ID NO: 62; LC was formed by fusing a VL sequence to a Cx chain of SEQ ID NO: 63. | | | | | | | | | | | | |

**Example 2. Production and Purification of Chimeric Antibodies**

(1) Expression in GS-CHO cells and purification:

According to the manufacturer's instructions, the GS Xceed™ Gene Expression System kit (Lonza) was used to produce an antibody-expressing CHO-S cell line. DNA sequences of the heavy and light chains of the antibody molecule were first inserted into the same pCHO1.0 plasmid, with the heavy chain being upstream of the light chain. The constructed pCHO1.0 plasmid was then transferred into the CHO cell line by chemical transfection and electrotransfection, and the antibody production was determined by ForteBio to determine the transfection efficiency after 48 h of transfection. The transfected cells were subjected to two cycles of pressurized filtration to obtain a cell pool with high antibody expression. The cells in the cell pool were then expanded to express a large number of antibodies, and the cell supernatant was collected and purified by Protein A affinity chromatography to achieve an antibody purity of > 95%.

(2) Expression in HEK293 cells and purification:

For a transient expression of an antibody in HEK293 cells, pcDNA3.1 vectors were used. cDNAs encoding the heavy and light chains of the antibody were first cloned into the pcDNA3.1 vectors. The vectors carrying the heavy and light chains of the antibody molecule were transferred into the HEK293 cells by chemical transfection. The cultured 293HEK was transiently transfected using a chemical transfection reagent PEI (purchased from Polysciences) according to a scheme provided by the manufacturer. After transfection, the medium was discarded and the cells were diluted to $4\times10^6$/mL with a fresh EXPI293 medium (Gibco). The cells were cultured at 37 °C with 5% $CO_2$ for 7 days, with the fresh medium fed every 48 h. After 7 days, the medium was centrifuged at 1300 rpm for 20 min. The supernatant was purified by Protein A affinity chromatography to achieve an antibody purity of > 95%.

**Example 3. Binding Kinetics of Chimeric Antibodies of the Present Invention for Antigens as Determined by Bio-Layer Interferometry**

In the present invention, the equilibrium dissociation constant ($K_D$) for the binding of the 3 exemplary chimeric antibodies of the present invention described above to human TNFR2 (hTNFR2), cynomolgus monkey TNFR2 (cTNFR2), murine TNFR2(mTNFR2), and human TNFR1(hTNFR1) was determined by biological optical interferometry (ForteBio). For comparison, the equilibrium dissociation constant ($K_D$) of a positive control antibody OPI (humanized antibody SBT-002e in WO 2017083525, VH sequence of SEQ ID NO: 58, VL sequence of SEQ ID NO: 59) was determined.
The ForteBio affinity assay was performed according to the prior art (Estep, P, et al., High throughput solution based measurement of antibody-antigen affinity and epitope binning. MAbs, 2013.5(2): p. 270-278). Briefly, a sensor was equilibrated offline in an assay buffer for 30 min, and was equilibrated online for 60 s to establish a baseline. The purified antibodies obtained as described above were loaded online onto an AHQ sensor (ForteBio) for the ForteBio affinity assay. The sensor with the loaded antibodies was then exposed to each of 100 nM hTNFR2, cTNFR2, mTNFR2, and hTNFR1 antigens for 5 min before transferring the sensor to an assay buffer for 5 min of dissociation for dissociation rate measurement. The kinetic analysis was performed using a 1:1 binding model.
In the experiment performed by the assay described above, the affinity values of 3C4, 69B1, 72G8, and the control antibody OPI are shown in Table 2.

Table 2. Affinity values of anti-TNFR2 antibodies

| Clone No. | $K_D$ (M) (the antibody is on the AMQ sensor chip, and rh-TNFR2-His (100 nM) is in the solution) | | | $K_D$(M) (the antibody is on the AMQ sensor chip, and CYNO-TNFR2-His (100 nM) is in the solution) | | |
|---|---|---|---|---|---|---|
| | $K_D$ (M) | $k_{on}$(1/Ms) | $k_{dis}$(1/s) | $K_D$ (M) | $k_{on}$(1/Ms) | $k_{dis}$(1/s) |
| 3C4 | 1.36E-09 | 1.29E+06 | 1.76E-03 | 1.42E-09 | 1.41E+06 | 2.01E-03 |
| 69B1 | 2.65E-10 | 1.96E+06 | 5.19E-04 | 1.11E-09 | 1.79E+06 | 1.98E-03 |
| OPI | 4.18E-09 | 5.48E+04 | 2.29E-04 | 1.15E-09 | 2.84E+05 | 3.28E-04 |
| 72G8 | 4.51E-09 | 1.14E+06 | 5.15E-03 | 3.11E-09 | 1.64E+06 | 5.09E-03 |

As can be seen from the results of the above tables, all of the above 4 antibodies showed very high affinity, among which 3C4, 69B1, and 72G8 had affinity comparable to or higher than the control antibody OPI.

**Example 4. Assay on Binding of Hybridoma Antibodies to CHO-S Cells Overexpressing Human TNFR2 (CHOS-hTNFR2) or CHO-S Cells Overexpressing Cynomolgus Monkey TNFR2 (CHOS-cTNFR2)**

In the study, the binding of the hybridoma antibodies (3C4, 69B1, and 72G8) of the present invention, after being diluted in a gradient, to a stable CHO-S cell strain overexpressing human TNFR2 on the surface was assayed by using a flow cytometer.

cDNAs encoding human TNFR2 (SEQ ID NO: 60, NM-001066.2) and cynomolgus monkey TNFR2 (SEQ ID NO: 61, XP-005544817.1) were cloned into pCHO1.0 vectors (Invitrogen), which were transfected into CHO-S cells (Invitrogen) to produce CHO-S cells overexpressing human TNFR2 (CHOS-hTNFR2) and CHO-S cells overexpressing cynomolgus monkey TNFR2 (CHOS-cTNFR2).

CHOS-hTNFR2 cells or CHOS-cTNFR2 cells were counted and diluted to $2 \times 10^6$ cells/mL, and added to a U-bottom 96-well plate at 100 $\mu$L/well. The mixture was centrifuged at 300 g for 5 min, and the cell medium was removed. The dilutions of the samples (the hybridoma antibodies 3C4, 69B1, and 72G8 and the positive control antibody OPI) (the antibodies were subjected to a two-fold serial dilution in PBS from the maximum antibody concentration of 300 nM) were added to the U-plate, and the cells were resuspended at 100 $\mu$L/well and left to stand on ice for 30 min. The suspension was centrifuged at 400 g for 5 min, and the supernatant was removed. The cells were washed twice with PBS. The mixture was centrifuged at 300 g for 5 min, and PBS was removed. 100 $\mu$L of anti-mouse IgG Alexa Fluor-488-labeled secondary antibody (Jackson ImmunoResearch; 115-545-072) (diluted at a 1:100 ratio in PBS) was added to each well and incubated on ice for 30 min in the dark. The mixture was centrifuged at 400 g for 5 min, and the supernatant was removed. The cells were washed 3 times with PBS. The cells were resuspended in 200 $\mu$L of 1$\times$ PBS and assayed by FACS.

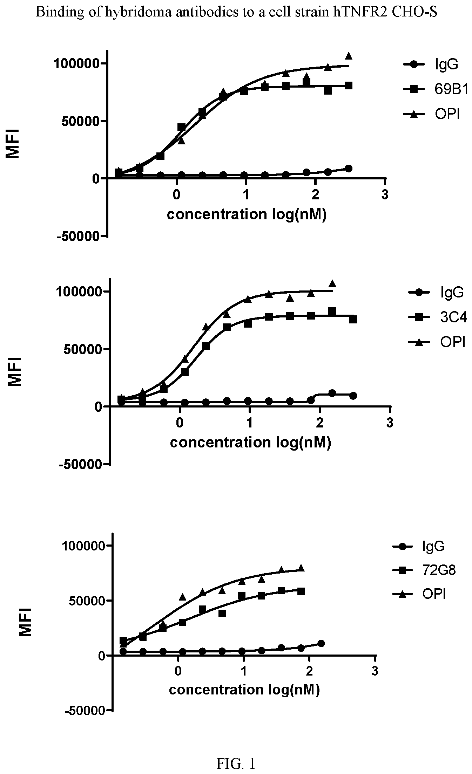

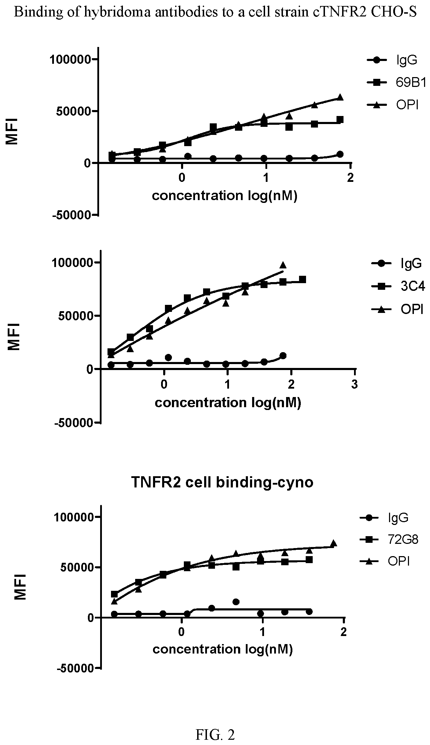

In the experiment performed by the assay described above, as shown in FIGs. 1 and 2, the results showed that the antibodies 3C4, 69B1, and 72G8 all bound to the human TNFR2 molecule (see FIG. 1) and cynomolgus monkey TNFR2 (see FIG. 2) overexpressed on CHO-S cells.

**Example 5. Assay on Blocking of Binding of TNFR2 to TNF$\alpha$ by Hybridoma Antibodies**

The ability of the hybridoma antibodies 3C4, 69B1, and 72G8 and the control antibody OPI to block the binding of human TNF$\alpha$ to hTNFR2 was assayed by ELISA.

The hTNFR2 protein was resuspended and dissolved to a concentration of 2 $\mu$g/mL with PBS, and a microplate was coated with the protein and incubated overnight. The plate was blocked with 5% BSA for 1 h, and a biotinylated antigen (Recombinant Biotinylated TNF$\alpha$ protein (ACRO)) was diluted to 3 $\mu$g/mL and added to the plate at 20 $\mu$L/well. The antibodies prepared as described above (3C4, 69B1, and 72G8) were each diluted in a gradient starting from the maximum concentration of 300 nM for a total of 8 or 12 dilution gradients. The diluted antibodies were added at 200 $\mu$L/well and incubated in PBS on ice for 30 min. The antigen-antibody mixture was incubated in a microplate for 90 min and washed three times with PBS, and the supernatant was discarded. 100 $\mu$L of Avidin-HRP (Invitrogen) diluted at a 1:5000 ratio was added to each well, incubated at room temperature for 30 min, and washed six times with PBS. A TMB chromogenic solution (solarbio) was added to the plate at 100 $\mu$L/well for color development for 1 min, and the reaction was stopped by adding a stop buffer (solarbio) at 100 $\mu$L/well. The plate was read on a microplate reader at $OD_{450}$ and $OD_{620}$.

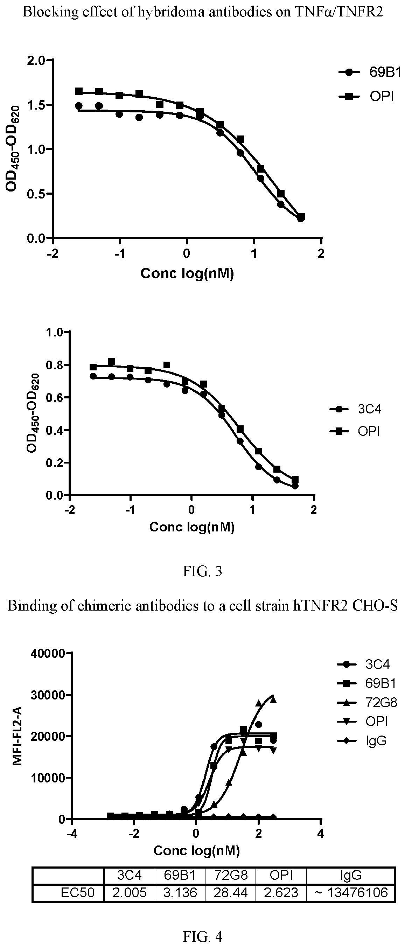

The experimental result showed that 72G8 had no blocking effect on the binding of the TNF$\alpha$ ligand to a receptor; while 3C4, 69B1, and the control antibody OPI had a complete blocking effect, and the $IC_{50}$ values for 3C4 and 69B1 were superior to that for OPI. (See FIG. 3)

**Example 6. Assay on Binding of Chimeric Antibodies to CHO-S Cells Overexpressing Human TNFR2 (CHOS-hTNFR2) or CHO-S Cells Overexpressing Cynomolgus Monkey TNFR2 (CHOS-cTNFR2)**

In the study, the binding of the chimeric antibodies of the present invention, after being diluted in a gradient, to a stable CHO-S cell strain overexpressing human TNFR2 on the surface was assayed by using a flow cytometer.

cDNAs encoding human TNFR2 (SEQ ID NO: 60) and cynomolgus monkey TNFR2 (SEQ ID NO: 61) were cloned into pCHO1.0 vectors (Invitrogen), which were transfected into CHO-S cells (Invitrogen) to produce CHO-S cells overexpressing human TNFR2 (CHOS-hTNFR2) and CHO-S cells overexpressing cynomolgus monkey TNFR2 (CHOS-cTNFR2).

CHOS-hTNFR2 cells or CHOS-cTNFR2 cells were counted and diluted to $2 \times 10^6$ cells/mL, and added to a U-bottom 96-well plate at 100 $\mu$L/well. The mixture was centrifuged at 300 g for 5 min, and the cell medium was removed. The dilutions of the samples (the chimeric antibodies 3C4, 69B1, and 72G8 and the positive control antibody OPI) (the antibodies were subjected to a three-fold serial dilution in PBS from the maximum antibody concentration of 300 nM) were added to the U-plate, and the cells were resuspended at 100 $\mu$L/well and left to stand on ice for 30 min. The suspension was centrifuged at 400 g for 5 min, and the supernatant was removed. The cells were washed twice with PBS. The mixture was centrifuged at 300 g for 5 min, and PBS was removed. 100 $\mu$L of anti-human Fc PE-labeled secondary antibody (SoutherBiotech; 2040-09) (diluted at a 1:100 ratio in PBS) was added to each well and incubated on ice for 30 min in the dark. The mixture was centrifuged at 400 g for 5 min, and the supernatant was removed. The cells were washed 3 times with PBS. The cells were resuspended in 200 $\mu$L of 1$\times$ PBS and assayed by FACS.

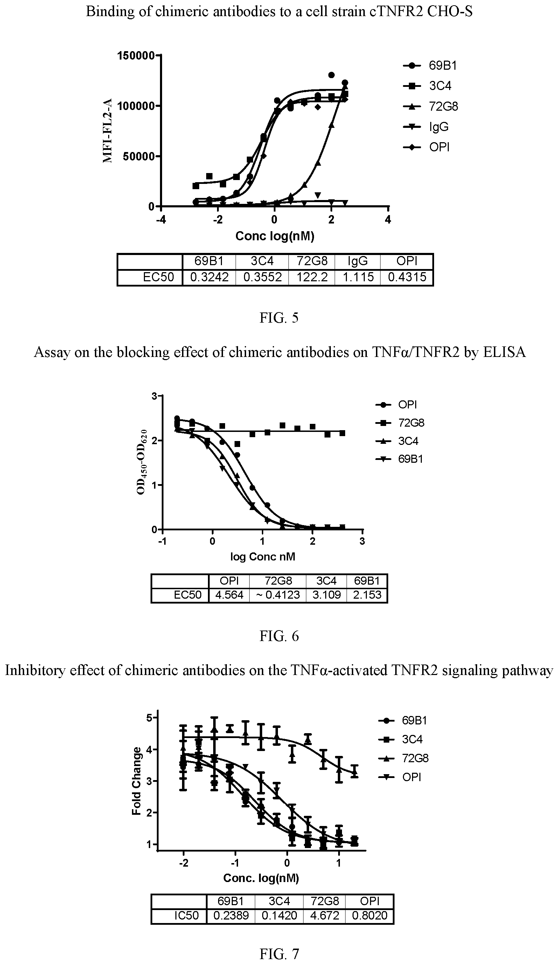

In the experiment performed by the assay described above, as shown in the figure, the results showed that the chimeric antibodies 3C4, 69B1, and 72G8 all bound to the human TNFR2 molecule overexpressed on CHO-S cells (see FIG. 4), and the antibodies 3C4, 69B1, and 72G8 all bound to cynomolgus monkey TNFR2 (see FIG. 5). The binding EC$_{50}$ values for the chimeric antibodies 3C4 and 69B1 were comparable to that of the positive control antibody OPI; but the binding of 72G8 was significantly weaker.

### Example 7. Assay on Blocking of Binding of TNFR2 to TNF$\alpha$ by Chimeric Antibodies

The ability of the chimeric antibodies 3C4, 69B1, and 72G8 and the control antibody OPI to block the binding of human TNF$\alpha$ to hTNFR2 was assayed by ELISA.

The hTNFR2 protein was resuspended and dissolved to a concentration of 2 $\mu$g/mL with PBS, and a microplate was coated with the protein and incubated overnight. The plate was blocked with 5% BSA for 1 h, and a biotinylated antigen (Recombinant Biotinylated TNF$\alpha$ protein (ACRO)) was diluted to 2 $\mu$g/mL and added to the plate at 10 $\mu$L/well. The antibodies prepared as described above (3C4, 69B1, and 72G8) were each diluted in a gradient starting from the maximum concentration of 300 nM for a total of 8 or 12 dilution gradients. The diluted antibodies were added at 100 $\mu$L/well and incubated in PBS on ice for 30 min. The antigen-antibody mixture was incubated in a microplate for 90 min and washed three times with PBS, and the supernatant was discarded. 100 $\mu$L of Avidin-HRP (Invitrogen) diluted at a 1:5000 ratio was added to each well, incubated at room temperature for 30 min, and washed six times with PBS. A TMB chromogenic solution (solarbio) was added to the plate at 100 $\mu$L/well for color development for 1 min, and the reaction was stopped by adding a stop buffer (solarbio) at 100 $\mu$L/well. The plate was read on a microplate reader at OD$_{450}$ and OD$_{620}$.

The experimental results showed that 3C4, 69B1, and the control antibody OPI had a complete blocking effect, while 72G8 has no blocking effect. The IC$_{50}$ values for the antibodies 3C4 and 69B1 were superior to that of Benchmark OPI. (See FIG. 6)

### Example 8. Assay on Inhibition of TNFR2-Mediated Activation of NF-$\kappa$B Signaling Pathway by Chimeric Antibodies

Inhibitory antibodies against TNFR2 can block the activation of the TNFR2 signaling pathway by a ligand TNF$\alpha$ by binding to TNFR2 on the cell membrane. In this study, the inhibitory effect of the antibodies of the present invention on the TNF$\alpha$-activated TNFR2 signaling pathway was investigated in Jurkat cells overexpressing TNFR2 by the Jurkat TNFR2 NF-$\kappa$B luciferase reporter system.

Assay steps:

Jurkat TNFR2 NF-$\kappa$B reporter cells were added to each well at $5\times10^4$ cells/well using 10% 1640 medium as a dilution buffer, and the cells were incubated with TNF$\alpha$ at a final concentration of 50 ng and different concentrations of antibody (maximum concentration of 20 nM, serial dilution at a 1:2 ratio) in a CO$_2$ incubator at 37 °C for 6 h. 100 $\mu$L of luciferase substrate solution was added to each well, and the plate was shaken for 2 min. The plate was read on a multimode microplate reader (Molecular Device i3). Fold change in antibody fluorescence readings was calculated according to the following equation:

Fold change = fluorescence reading of each well to which antibody was added/fluorescence reading of the control group (treatment group without TNF$\alpha$).

The experimental results (FIG. 7) showed that the chimeric antibodies 3C4 and 69B1 blocking the binding of TNFR2 to TNF$\alpha$ both could inhibit the TNF$\alpha$-induced activation of the TNFR2 NF-$\kappa$B signaling pathway, and the antibodies 3C4 and 69B1 had better inhibitory activity than the positive control antibody OPI. Furthermore, surprisingly, the antibody 72G8 exhibited a novel mechanism of inhibition of TNFR2 functional activity, by which the antibody could directly inhibit the polymerization of TNFR2 without blocking the binding of TNFR2 to TNF$\alpha$, thereby inhibiting the TNF$\alpha$-induced activation of the TNFR2 NF-$\kappa$B signaling pathway.

**Example 9. Humanization of Chimeric Antibodies**

The resulting chimeric antibodies 3C4 and 69B1 were humanized. The steps were as follows:

(1) determining a CDR loop structure;
(2) searching a human germline sequence database for the closest homologous sequences for each V/J region of the heavy and light chains;
(3) screening the human germlines for the highest match in heavy and light chains and a minimum quantity of mutations;
(4) constructing the CDRs of the chimeric antibody onto the framework of a human antibody;
(5) determining the positions of amino acids that maintained the CDR functions in the framework based on the sequences and structural features;
(6) adding back mutations (back to the input amino acids) at important positions identified; and
(7) optimizing amino acids at risk sites.

The amino acid sequences of the CDRs, the light and heavy chain variable regions, and the light and heavy chains of the obtained humanized antibodies hz3C4.7, hz69B1.5, hz69B1.6, hz69B1.11, and hz69B1.20 are shown in the attached sequence listing.

**Example 10. Assay on the Binding of Humanized Antibodies to Cells Expressing Human TNFR2**

In the study, the binding of the antibodies, after being diluted in a gradient, to a stable CHO-S strain line overexpressing human TNFR2 on the surface was assayed by using a flow cytometer.

cDNA encoding human TNFR2 was cloned into a pCHO1.0 vector (Invitrogen), which was transfected into CHO-S cells (Invitrogen) to produce CHO-S cells overexpressing human TNFR2 (CHOS-hTNFR2).

CHOS-hTNFR2 cells were counted and diluted to $2\times10^6$ cells/mL, and added to a U-bottom 96-well plate at 100 $\mu$L/well. The mixture was centrifuged at 300 g for 5 min, and the cell medium was removed. Samples (the humanized antibodies hz3C4.7, hz69B1.5, hz69B1.6, hz69B1.11 and hz69B1.20, the chimeric antibody 69B1, and the positive control antibody OPI) (the antibodies were subjected to a three-fold dilution in PBS from the maximum antibody concentration of 300 nM) were added to the U-plate, and the cells were resuspended at 100 $\mu$L/well and left to stand on ice for 30 min. The suspension was centrifuged at 400 g for 5 min, and the supernatant was removed. The cells were washed twice with PBS. The mixture was centrifuged at 300 g for 5 min, and PBS was removed. 100 $\mu$L of anti-human Fc PE-labeled secondary antibody (SoutherBiotech; 2040-09) (diluted at a 1:100 ratio in PBS) was added to each well and incubated on ice for 30 min in the dark. The mixture was centrifuged at 400 g for 5 min, and the supernatant was removed. The cells were washed 3 times with PBS. The cells were resuspended in 200 $\mu$L of 1$\times$ PBS and assayed by FACS.

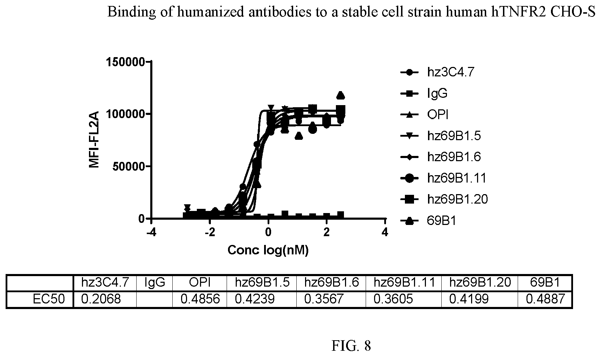

In the experiment performed by the assay described above, the binding of the antibodies to CHOS-hTNFR2 cells is shown in FIG. 8. The humanized antibodies hz3C4.7, hz69B1.5, hz69B1.6, hz69B1.11, and hz69B1.20 all bound to a human TNFR2 molecule overexpressed on CHO-S cells (see FIG. 8).

**Example 11. Assay on Blocking of Binding of TNFR2 to TNF$\alpha$ by Humanized Antibodies**

In a similar manner to that in Example 7, the ability of the humanized antibodies hz3C4.7, hz69b1.5, hz69b1.6, hz69b1.11, and hz69b1.20, and the control antibody OPI to block the binding of human TNF$\alpha$ to hTNFR2 was assayed by ELISA.

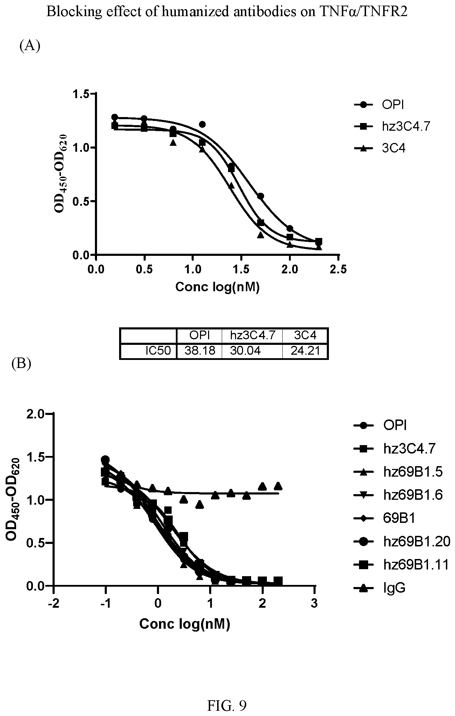

The results are shown in FIGs. 9Aand 9B. As shown in the figures, the humanized forms of 3C4 and 69B1 exhibited complete blocking effects.

**Example 12. Assay on Inhibition of TNFR2-Mediated Activation of NF-$\kappa$B Signaling Pathway by Humanized Antibodies**

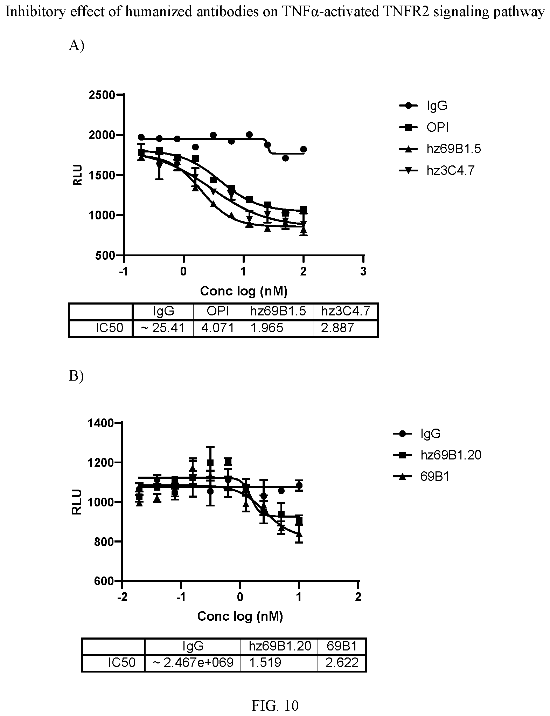

In a similar manner as that in Example 8, the inhibitory effect of humanized forms of antibodies 3C4 and 69B1 on the TNFR2 signaling pathway activated by TNF$\alpha$ was investigated in Jurkat cells overexpressing TNFR2 by the Jurkat TNFR2 NF-$\kappa$B luciferase reporter system. The results are shown in FIGs. 10A and 10B.

**Example 13. Assay on the Binding of Humanized Antibodies to K562 and Treg Cells**

In this study, the binding of the humanized antibodies of the present invention, after being diluted in a gradient, to K562 cells (ATCC, chronic myeloid leukemia cells) and Treg cells by using a flow cytometer.

Tregs were prepared as follows: Human PBMC cells (ALLCELLS, PB005F) were thawed, $CD4^+ CD25^+ CD127^{low}$ cells were sorted by flow cytometry, and Treg cells were isolated. According to $CD4^+$:anti-CD3/CD28 Beads = 1:1, Dynabeads Human T-Activator CD3/CD28 (Gibco, 11131D) was added, followed by 10 ng/mL recombinant IL2, and the cells were stimulated for 7 days.

Assay steps: The mixture was centrifuged at 400 g for 5 min, and the cell medium was removed. K562 or Treg cells were resuspended in PBS. After counting, the cells were adjusted to a density of $2 \times 10^6$/mL, and the cell suspension was added to a U-bottom 96-well plate at 100 μL/well. The test antibody was added, subjected to a three-fold gradient dilution, and left to stand on ice for 30 min. The mixture was centrifuged at 300 g for 5 min, and the supernatant was removed. The cells were washed once with PBS. The mixture was centrifuged at 300 g for 5 min, and PBS was removed. 100 μL of PE-anti-human Fc antibody (SOUTHERN BIOTECH, 2040-09) diluted at a ratio of 1:200 was added to each well. The mixture was incubated on ice for 30 min in the dark. The mixture was centrifuged at 400 g for 5 min, and the supernatant was removed. The cells were washed twice with PBS. The cells were resuspended in 100 μL of PBS and assayed by using a flow cytometer (BD, ACCURIC6 plus).

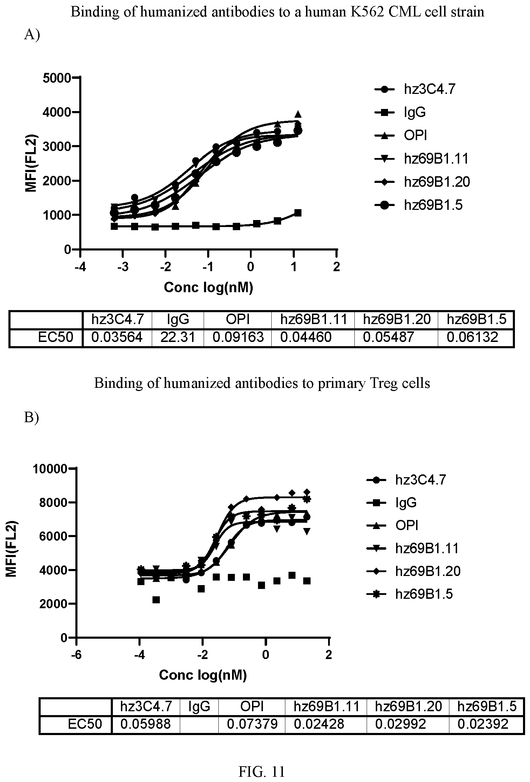

In the above experiment, as shown in FIG. 11, the humanized antibodies hz3C4.7, hz69B1.5, hz69B1.11, and hz69B1.20 had superior binding ability to K562 and Treg cells to the control antibody OPI.

## Example 14. ADCC Reporter Assay on Humanized Antibodies

In this study, the ADCC killing effect of the humanized antibodies of the present invention, after being diluted in a gradient, on CHOS-hTNFR2 cells was assayed by the Jurkat ADCC reporter assay (Promega, G7102).

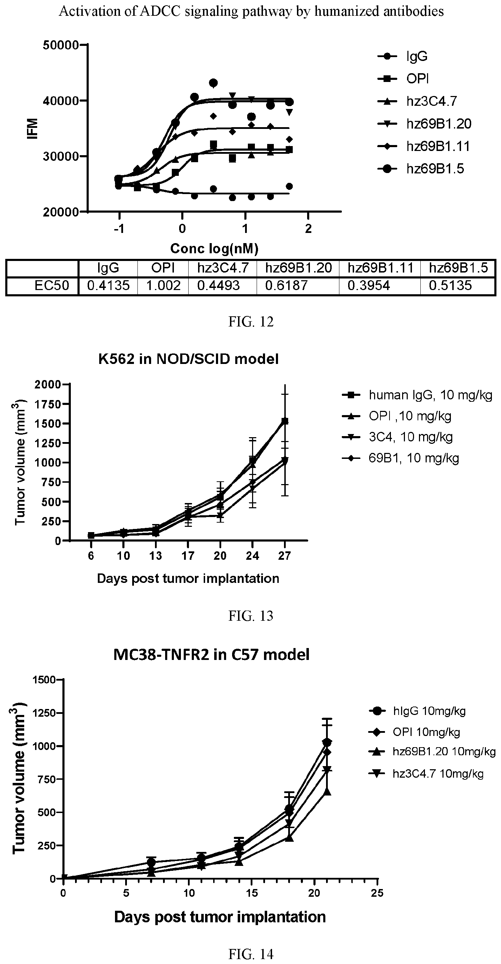

Assay steps: The antibodies were subjected to a serial dilution at a 1:2 ratio starting from 50 nM according to the protocol supplied by the supplier. Then, $2.5 \times 10^4$ CHOS-hTNFR2 cells, and $1.75 \times 10^5$ Jurkat effector cells were added to each well. The mixture was incubated in a $CO_2$ incubator at 37 °C for 20 h, and a luciferase substrate was added. After 2 min, the plate was read on a multimode microplate reader (MD, I3 multimode microplate reader). In the above experiment, as shown in FIG. 12, the humanized antibodies hz3C4.7, hz69B1.5, hz69B1.11, and hz69B1.20 had a better ADCC killing effect on CHO-S cells expressing hTNFR2 than the control antibody OPI.

## Example 15. Anti-K562 Tumor Efficacy Assay

In the experiment, NOD/SCID mice were inoculated with K562 cells for the determination of the anti-tumor effect of the anti-TNFR2 antibodies of the present invention.

NOD-SCID mice:

SPF female NOD/SCID mice, purchased from Beijing Vital River Laboratory Animal Technology Co., Ltd., qualified by Beijing Vital River Laboratory Animal Technology Co., Ltd., with certificate No. 11400700380384. The study started after the mice were acclimated for 7 days after arrival.

Cells:

K562 cells were human chronic myeloid leukemia cells purchased from ATCC (CAT#: CCL-243) and were routinely subcultured strictly according to the instructions for subsequent *in vivo* experiments. The cells were collected by centrifugation, resuspended in Matrigel, and adjusted to a density of $5 \times 10^6$ cells/mL. 0.2 mL of the cell suspension was subcutaneously inoculated into the right abdominal region of each of the NOD-SCID mice to establish K562 tumor-bearing mouse models.

Administration:

Tumor volumes of the mice were measured 6 days after tumor cell inoculation, and the mice with tumor volumes in the range of 25 $mm^3$-118 $mm^3$ were selected and randomly grouped (8 mice in each group) according to tumor volume, with the average tumor volume of 65 $mm^3$ per group. The dosage and mode of administration are shown in Table 3. h-IgG (purchased from EQUITECH-BIO) was used as a negative control. The mice were subjected to administration on day 6, day 10, day 13, and day 17 after inoculation, twice a week, for a total of four doses. The tumor volume and the body weight of the mice were monitored. The body weight and tumor volume were measured before each administration. On day 27 after inoculation, the relative tumor growth inhibition (TGI%) was calculated by the following formula: TGI% = 100% ×(control group tumor volume - treatment group tumor volume)/(control group tumor volume - control group tumor volume before administration). Tumor volume measurement: The maximum length of major axis (L) and maximum length of minor axis (W) of tumors were measured with a vernier caliper, and tumor volume was calculated by the following formula: $V = L \times W^2/2$. The body weight was measured using an electronic balance.

Table 3. Experimental design

| Group | Dosage of administration | Administration frequency | Route of administration |
|---|---|---|---|
| h-IgG | 10 mg/kg | Q3-4Dx4 | Intraperitoneal injection |
| OPI | 10 mg/kg | Q3-4Dx4 | Intraperitoneal injection |
| 3C4 | 10 mg/kg | Q3-4Dx4 | Intraperitoneal injection |
| 69B1 | 10 mg/kg | Q3-4Dx4 | Intraperitoneal injection |

As shown in FIG. 13 and Table 4, the results for the tumor growth inhibition showed that on day 27 after inoculation, the chimeric antibodies 3C4 and 69B1, when used alone, both showed a tumor inhibitory effect. The tumor growth inhibition was 37% for 3C4 and 33% for 69B1. Therefore, the antibodies against the TNFR2 molecule of the present invention have an inhibitory effect on tumors.

Table 4. Tumor growth inhibition on Day 27

| Group | Tumor volume ($mm^3$) | TGI(%) |
|---|---|---|
| h-IgG | 1529 | / |
| OPI | 1555 | None |
| 3C4 | 995 | 37 |
| 69B1 | 1043 | 33 |

**Example 16. Anti-MC38-TNFR2 Tumor Efficacy Assay**

In the experiment, C57BL/6 mice were inoculated with MC38-TNFR2 cells (mouse colon cancer cells overexpressing human TNFR2) for the determination of the anti-tumor effect of the anti-TNFR2 antibodies of the present invention.

C57 mice:

SPF male C57BL/6 mice, purchased from Beijing Vital River Laboratory Animal Technology Co., Ltd., qualified by Beijing Vital River Laboratory Animal Technology Co., Ltd., with certificate No. 1100111911055796. The study started after the mice were acclimated for 7 days after arrival.

Cells:

Mouse MC38-TNFR2 cells were constructed in-house and routinely subcultured strictly according to the instructions for subsequent *in vivo* experiments. The cells were collected by centrifugation, resuspended in PBS, and adjusted to a density of $2 \times 10^6$ cells/mL. On day 0, 0.2 mL of the cell suspension was subcutaneously inoculated into the right abdominal region of the human C57BL/6 mice to establish MC38-TNFR2 tumor-bearing mouse models.

Administration:

Tumor volumes of the mice were measured 7 days after tumor cell inoculation, and the mice were randomly grouped (7 mice in each group) according to tumor volume, with the average tumor volume of 63 $mm^3$ per group. The dosage and mode of administration are shown in Table 5. h-IgG (purchased from EQUITECH-BIO) was used as a negative control. The mice were subjected to administration twice a week for a total of four doses. The tumor volume and the body weight of mice were monitored. On day 21 after inoculation, the relative tumor growth inhibition (TGI%) was calculated by the following formula: TGI% = 100% $\times$(control group tumor volume - treatment group tumor volume)/(control group tumor volume - control group tumor volume before administration). Tumor volume measurement: The maximum length of major axis (L) and maximum length of minor axis (W) of tumors were measured with a vernier caliper, and tumor volume was calculated by the following formula: $V = L \times W^2/2$. The body weight was measured using an electronic balance.

Table 5. Experimental design

| Group | Dosage of administration | Administration frequency | Route of administration |
| --- | --- | --- | --- |
| h-IgG | 10 mg/kg | Q3-4Dx4 | Intraperitoneal injection |
| OPI | 10 mg/kg | Q3-4Dx4 | Intraperitoneal injection |
| hz69B1.20 | 10 mg/kg | Q3-4Dx4 | Intraperitoneal injection |
| hz3C4.7 | 10 mg/kg | Q3-4Dx4 | Intraperitoneal injection |

As shown in FIG. 14 and Table 6, the results for the tumor growth inhibition showed that on day 21 after inoculation, hz3C4.7 and hz69B1.20, when used alone, both showed a tumor inhibitory effect. The single-drug tumor growth inhibition was 28% for Hz3C4.7 and 41% for hz69B1.20. Therefore, the antibodies against the TNFR2 molecule of the present invention have an inhibitory effect on tumors.

Table 5. Tumor growth inhibition on Day 21

| Group | Tumor volume ($mm^3$) | TGI(%) |
| --- | --- | --- |
| h-IgG | 1029 | / |
| OPI | 954 | 8 |
| hz3C4.7 | 815 | 28 |
| hz69B1.20 | 660 | 41 |

**Claims**

1. An antibody or an antigen-binding fragment thereof binding to TNFR2, comprising

(i) HCDR1, HCDR2, and HCDR3 sequences of a heavy chain variable region set forth in SEQ ID NO: 19, and LCDR1, LCDR2, and LCDR3 sequences of a light chain variable region set forth in SEQ ID NO: 20, or
(ii) HCDR1, HCDR2, and HCDR3 sequences of a heavy chain variable region set forth in SEQ ID NO: 21 and LCDR1, LCDR2, and LCDR3 sequences of a light chain variable region set forth in SEQ ID NO: 22, or
(iii) HCDR1, HCDR2, and HCDR3 sequences of a heavy chain variable region set forth in SEQ ID NO: 23 and LCDR1, LCDR2, and LCDR3 sequences of a light chain variable region set forth in SEQ ID NO:24, or
(iv) HCDR1, HCDR2, and HCDR3 sequences of a heavy chain variable region set forth in SEQ ID NO: 25 and LCDR1, LCDR2, and LCDR3 sequences of a light chain variable region set forth in SEQ ID NO: 26, or
(v) HCDR1, HCDR2, and HCDR3 sequences of a heavy chain variable region set forth in SEQ ID NO: 27 and LCDR1, LCDR2, and LCDR3 sequences of a light chain variable region set forth in SEQ ID NO: 28, or
(vi) HCDR1, HCDR2, and HCDR3 sequences of a heavy chain variable region set forth in SEQ ID NO:29 and LCDR1, LCDR2, and LCDR3 sequences of a light chain variable region set forth in SEQ ID NO: 30 or 31, or
(vii) HCDR1, HCDR2, and HCDR3 sequences of a heavy chain variable region set forth in SEQ ID NO: 64, and LCDR1, LCDR2, and LCDR3 sequences of a light chain variable region set forth in SEQ ID NO: 65.

2. An antibody or an antigen-binding fragment thereof binding to TNFR2, comprising three complementarity determining regions (HCDRs) of a heavy chain variable region and three complementarity determining regions (LCDRs) of a light chain variable region, wherein:

(i) HCDR1 comprises or consists of an amino acid sequence of SEQ ID NO: 1, HCDR2 comprises or consists of an amino acid sequence of SEQ ID NO: 2, HCDR3 comprises or consists of an amino acid sequence of SEQ ID NO: 3, LCDR1 comprises or consists of an amino acid sequence of SEQ ID NO: 4, LCDR2 comprises or consists of an amino acid sequence of SEQ ID NO: 5, and LCDR3 comprises or consists of an amino acid sequence of SEQ ID NO: 6; or
(ii) HCDR1 comprises or consists of an amino acid sequence of SEQ ID NO: 7, HCDR2 comprises or consists of an amino acid sequence of SEQ ID NO: 8, HCDR3 comprises or consists of an amino acid sequence of SEQ ID NO: 9, LCDR1 comprises or consists of an amino acid sequence of SEQ ID NO: 10, LCDR2 comprises or consists of an amino acid sequence of SEQ ID NO: 11, and LCDR3 comprises or consists of an amino acid sequence of SEQ ID NO: 12; or
(iii) HCDR1 comprises or consists of an amino acid sequence of SEQ ID NO: 13, HCDR2 comprises or consists of an amino acid sequence of SEQ ID NO: 14, HCDR3 comprises or consists of an amino acid sequence of SEQ ID NO: 15, LCDR1 comprises or consists of an amino acid sequence of SEQ ID NO: 16, LCDR2 comprises or consists of an amino acid sequence of SEQ ID NO: 17, and LCDR3 comprises or consists of an amino acid sequence of SEQ ID NO: 18;

or wherein the antibody comprises a variant of one of the CDR sequence combinations of (i)-(iii), wherein the variant comprises at least one and no more than 5, 4, 3, 2, or 1 amino acid change (preferably amino acid substitution, preferably conservative substitution) in total in terms of 6 CDR regions, and preferably, the heavy chain CDR3 remains unchanged.

3. The antibody or the antigen-binding fragment thereof according to claim 1 or 2, comprising a heavy chain variable region and a light chain variable region, wherein:
the heavy chain variable region comprises:

(i) an amino acid sequence set forth in SEQ ID NO: 19, or an amino acid sequence having at least 80%, 85%, 90%, 95%, 96%, 97%, 98%, or 99% sequence identity thereto, or
(ii) an amino acid sequence set forth in SEQ ID NO: 21, or an amino acid sequence having at least 80%, 85%, 90%, 95%, 96%, 97%, 98%, or 99% sequence identity thereto, or
(iii) an amino acid sequence set forth in SEQ ID NO: 23, or an amino acid sequence having at least 80%, 85%, 90%, 95%, 96%, 97%, 98%, or 99% sequence identity thereto, or
(iv) an amino acid sequence set forth in SEQ ID NO: 25, or an amino acid sequence having at least 80%, 85%, 90%, 95%, 96%, 97%, 98%, or 99% sequence identity thereto, or
(v) an amino acid sequence set forth in SEQ ID NO: 27, or an amino acid sequence having at least 80%, 85%, 90%, 95%, 96%, 97%, 98%, or 99% sequence identity thereto, or
(vi) an amino acid sequence set forth in SEQ ID NO: 29, or an amino acid sequence having at least 80%, 85%, 90%, 95%, 96%, 97%, 98%, or 99% sequence identity thereto, or
(vii) an amino acid sequence set forth in SEQ ID NO: 64, or an amino acid sequence having at least 80%, 85%, 90%, 95%, 96%, 97%, 98%, or 99% sequence identity thereto.

4. The antibody or the antigen-binding fragment thereof according to claim 1 or 2, comprising a heavy chain variable region and a light chain variable region, wherein:
the light chain variable region comprises:

(i) an amino acid sequence set forth in SEQ ID NO: 20, or an amino acid sequence having at least 80%, 85%, 90%, 95%, 96%, 97%, 98%, or 99% sequence identity thereto, or
(ii) an amino acid sequence set forth in SEQ ID NO: 22, or an amino acid sequence having at least 80%, 85%, 90%, 95%, 96%, 97%, 98%, or 99% sequence identity thereto, or
(iii) an amino acid sequence set forth in SEQ ID NO: 24, or an amino acid sequence having at least 80%, 85%, 90%, 95%, 96%, 97%, 98%, or 99% sequence identity thereto, or
(iv) an amino acid sequence set forth in SEQ ID NO: 26, or an amino acid sequence having at least 80%, 85%, 90%, 95%, 96%, 97%, 98%, or 99% sequence identity thereto, or
(v) an amino acid sequence set forth in SEQ ID NO: 28, or an amino acid sequence having at least 80%, 85%, 90%, 95%, 96%, 97%, 98%, or 99% sequence identity thereto, or
(vi) an amino acid sequence set forth in SEQ ID NO: 30 or 21, or an amino acid sequence having at least 80%, 85%, 90%, 95%, 96%, 97%, 98%, or 99% sequence identity thereto, or
(vii) an amino acid sequence set forth in SEQ ID NO: 65, or an amino acid sequence having at least 80%, 85%, 90%, 95%, 96%, 97%, 98%, or 99% sequence identity thereto.

5. The antibody or the antigen-binding fragment thereof according to any one of claims 1 to 4, comprising a heavy chain variable region and a light chain variable region selected from:

(i) a heavy chain variable region comprising an amino acid sequence set forth in SEQ ID NO: 19 and a light chain variable region comprising an amino acid sequence set forth in SEQ ID NO: 20, or
(ii) a heavy chain variable region comprising an amino acid sequence set forth in SEQ ID NO: 21 and a light chain variable region comprising an amino acid sequence set forth in SEQ ID NO: 22, or
(iii) a heavy chain variable region comprising an amino acid sequence set forth in SEQ ID NO: 23, and a light chain variable region comprising an amino acid sequence set forth in SEQ ID NO: 24, or
(iv) a heavy chain variable region comprising an amino acid sequence set forth in SEQ ID NO: 25 and a light chain variable region comprising an amino acid sequence set forth in SEQ ID NO: 26, or

(v) a heavy chain variable region comprising an amino acid sequence set forth in SEQ ID NO: 27 and a light chain variable region comprising an amino acid sequence set forth in SEQ ID NO: 28, or

(vi) a heavy chain variable region comprising an amino acid sequence set forth in SEQ ID NO: 29 and a light chain variable region comprising an amino acid sequence set forth in SEQ ID NO: 30 or 31, or

(vii) a heavy chain variable region comprising an amino acid sequence set forth in SEQ ID NO: 64 and a light chain variable region comprising an amino acid sequence set forth in SEQ ID NO: 65.

6. The antibody or the antigen-binding fragment thereof according to any one of the preceding claims, wherein the antibody is an antibody in the form of IgG1, IgG2, IgG3 or IgG4 or an antigen-binding fragment thereof, preferably a human IgG1 Fc region, and preferably, the antibody induces ADCC activity in the presence of effector cells, such as NK cells.

7. The antibody or the antigen-binding fragment thereof according to any one of the preceding claims, wherein the antibody is a murine antibody, a chimeric antibody, a humanized antibody, or a fully human antibody, and the antigen-binding fragment is an antibody fragment selected from: a Fab, a Fab', a Fab'-SH, an Fv, a single-chain antibody, an scFv, an (Fab')$_2$ fragment, a single-domain antibody, a diabody (dAb), and a linear antibody.

8. An anti-TNFR2 antibody or an antigen-binding fragment thereof, wherein the antibody has one or more of the following properties:

(i) inhibiting (e.g., competitively inhibiting) the binding of any one of the antibodies according to claims 1-7 to human TNFR2;
(ii) binding to the same or overlapping epitope as any one of the antibodies according to claims 1-7; and
(iii) competing with any one of the antibodies according to claims 1-7 for binding to human TNFR2.

9. An isolated nucleic acid encoding the anti-TNFR2 antibody or the antigen-binding fragment thereof according to any one of the preceding claims.

10. A vector comprising the nucleic acid according to claim 9, wherein preferably, the vector is an expression vector.

11. A host cell comprising the nucleic acid according to claim 9 or the vector according to claim 10, wherein preferably, the host cell is a mammalian cell.

12. A method for preparing an anti-TNFR2 antibody or an antigen-binding fragment thereof, comprising culturing a host cell comprising a nucleic acid encoding the antibody or the antigen-binding fragment thereof according to any one of claims 1-8 under conditions suitable for expressing the nucleic acid, and optionally isolating the antibody or the antigen-binding fragment thereof, wherein optionally, the method further comprises recovering the anti-TNFR2 antibody or the antigen-binding fragment thereof from the host cell.

13. An immunoconjugate comprising the antibody or the antigen-binding fragment thereof according to any one of claims 1-8 conjugated to a therapeutic or diagnostic agent.

14. A multispecific antibody comprising the antibody or the antigen-binding fragment thereof according to any one of claims 1-8.

15. A pharmaceutical composition comprising the antibody or the antigen-binding fragment thereof according to any one of claims 1-8, the immunoconjugate according to claim 13, or the multispecific antibody according to claim 14, and optionally a pharmaceutical auxiliary material.

16. Use of the antibody or the antigen-binding fragment thereof according to any one of claims 1-8, *in vivo* or *in vitro,* for purposes selected from:

(1) blocking the binding of TNFR-2 to its ligand TNFα;
(2) directly inhibiting polymerization of TNFR2 without blocking the binding of TNFR-2 to its ligand TNFα;
(3) inhibiting activation of a TNFR2 receptor in a manner dependent on or independent of blocking of the binding of TNFα to TNFR2;
(4) inhibiting activation of NF-xB signaling pathway mediated by the binding of TNFα to TNFR2;
(5) antagonizing a Treg cell proliferation promoting effect of TNFα; and/or

(6) depending on ADCC activity elicited by an antibody Fc region, killing tumor cells expressing TNFR2 on the surface.

17. A method for preventing or treating a tumor in a subject, comprising administering to the subject an effective amount of the anti-TNFR2 antibody or the antigen-binding fragment thereof according to any one of claims 1-8, the immunoconjugate according to claim 13, the multispecific antibody according to claim 14, or the pharmaceutical composition according to claim 15, wherein preferably, the tumor is colon cancer or chronic myeloid leukemia.

18. A method for detecting TNFR2 in a sample, comprising

(a) contacting the sample with the antibody or the antigen-binding fragment thereof according to any one of claims 1-8; and
(b) detecting a complex formed by the antibody or the antigen-binding fragment thereof with TNFR2, wherein optionally, the antibody is detectably labeled.

Binding of hybridoma antibodies to a cell strain hTNFR2 CHO-S

FIG. 1

Binding of hybridoma antibodies to a cell strain cTNFR2 CHO-S

TNFR2 cell binding-cyno

FIG. 2

Blocking effect of hybridoma antibodies on TNFα/TNFR2

FIG. 3

Binding of chimeric antibodies to a cell strain hTNFR2 CHO-S

| | 3C4 | 69B1 | 72G8 | OPI | IgG |
|---|---|---|---|---|---|
| EC50 | 2.005 | 3.136 | 28.44 | 2.623 | ~ 13476106 |

FIG. 4

Binding of chimeric antibodies to a cell strain cTNFR2 CHO-S

| | 69B1 | 3C4 | 72G8 | IgG | OPI |
|---|---|---|---|---|---|
| EC50 | 0.3242 | 0.3552 | 122.2 | 1.115 | 0.4315 |

FIG. 5

Assay on the blocking effect of chimeric antibodies on TNFα/TNFR2 by ELISA

| | OPI | 72G8 | 3C4 | 69B1 |
|---|---|---|---|---|
| EC50 | 4.564 | ~ 0.4123 | 3.109 | 2.153 |

FIG. 6

Inhibitory effect of chimeric antibodies on the TNFα-activated TNFR2 signaling pathway

| | 69B1 | 3C4 | 72G8 | OPI |
|---|---|---|---|---|
| IC50 | 0.2389 | 0.1420 | 4.672 | 0.8020 |

FIG. 7

Binding of humanized antibodies to a stable cell strain human hTNFR2 CHO-S

| | hz3C4.7 | IgG | OPI | hz69B1.5 | hz69B1.6 | hz69B1.11 | hz69B1.20 | 69B1 |
|---|---|---|---|---|---|---|---|---|
| EC50 | 0.2068 | | 0.4856 | 0.4239 | 0.3567 | 0.3605 | 0.4199 | 0.4887 |

FIG. 8

Blocking effect of humanized antibodies on TNFα/TNFR2

(A)

| | OPI | hz3C4.7 | 3C4 |
|---|---|---|---|
| IC50 | 38.18 | 30.04 | 24.21 |

(B)

FIG. 9

Inhibitory effect of humanized antibodies on TNFα-activated TNFR2 signaling pathway

A)

|  | IgG | OPI | hz69B1.5 | hz3C4.7 |
|---|---|---|---|---|
| IC50 | ~ 25.41 | 4.071 | 1.965 | 2.887 |

B)

|  | IgG | hz69B1.20 | 69B1 |
|---|---|---|---|
| IC50 | ~ 2.467e+069 | 1.519 | 2.622 |

FIG. 10

Binding of humanized antibodies to a human K562 CML cell strain

A)

| | hz3C4.7 | IgG | OPI | hz69B1.11 | hz69B1.20 | hz69B1.5 |
|---|---|---|---|---|---|---|
| EC50 | 0.03564 | 22.31 | 0.09163 | 0.04460 | 0.05487 | 0.06132 |

Binding of humanized antibodies to primary Treg cells

B)

| | hz3C4.7 | IgG | OPI | hz69B1.11 | hz69B1.20 | hz69B1.5 |
|---|---|---|---|---|---|---|
| EC50 | 0.05988 | | 0.07379 | 0.02428 | 0.02992 | 0.02392 |

FIG. 11

Activation of ADCC signaling pathway by humanized antibodies

| | IgG | OPI | hz3C4.7 | hz69B1.20 | hz69B1.11 | hz69B1.5 |
|---|---|---|---|---|---|---|
| EC50 | 0.4135 | 1.002 | 0.4493 | 0.6187 | 0.3954 | 0.5135 |

FIG. 12

K562 in NOD/SCID model

FIG. 13

MC38-TNFR2 in C57 model

FIG. 14

CDR sequences of exemplary chimeric antibodies and humanized antibodies

| Antibody | Heavy chain CDR sequence | | | | | | |
|---|---|---|---|---|---|---|---|
| name | HCDR1 | SEQ ID | HCDR2 | SEQ ID | HCDR3 | SEQ ID |
| 3C4 | DYSFTNYYIH | 1 | WIYPGNVITKYHEKFKG | 2 | SRATGYFDY | 3 |
| hz3C4.7 | DYSFTNYYIH | 1 | WIYPGNVITKYHEKFKG | 2 | SRATGYFDY | 3 |
| 72G8 | GYTFTSYWMQ | 7 | AIYPGDGDTRYTQKFKG | 8 | DDGLYYAMDY | 9 |
| 69B1 | GYTFTDYSMN | 13 | WLNTETGEPIYADDFKG | 14 | EAIYYGTSYVLDY | 15 |
| hz69B1.5 | GYTFTDYSMN | 13 | WLNTETGEPIYADDFKG | 14 | EAIYYGTSYVLDY | 15 |
| hz69B1.6 | GYTFTDYSMN | 13 | WLNTETGEPIYADDFKG | 14 | EAIYYGTSYVLDY | 15 |
| hz69B1.11 | GYTFTDYSMN | 13 | WLNTETGEPIYADDFKG | 14 | EAIYYGTSYVLDY | 15 |
| hz69B1.20 | GYTFTDYSMN | 13 | WLNTETGEPIYADDFKG | 14 | EAIYYGTSYVLDY | 15 |

| Antibody | Light chain CDR sequence | | | | | | |
|---|---|---|---|---|---|---|---|
| name | LCDR1 | SEQ ID | LCDR2 | SEQ ID | LCDR3 | SEQ ID |
| 3C4 | RASQDIRNYLN | 4 | YTSRLHS | 5 | QQFNTLPWT | 6 |
| hz3C4.7 | RASQDIRNYLN | 4 | YTSRLHS | 5 | QQFNTLPWT | 6 |
| 72G8 | TASSSVSSSYLH | 10 | STSNLAS | 11 | HQYHRSPPT | 12 |
| 69B1 | RASESVEYYGTNFMQ | 16 | SASNVES | 17 | QQSRKDPST | 18 |
| hz69B1.5 | RASESVEYYGTNFMQ | 16 | SASNVES | 17 | QQSRKDPST | 18 |
| hz69B1.6 | RASESVEYYGTNFMQ | 16 | SASNVES | 17 | QQSRKDPST | 18 |
| hz69B1.11 | RASESVEYYGTNFMQ | 16 | SASNVES | 17 | QQSRKDPST | 18 |
| hz69B1.20 | RASESVEYYGTNFMQ | 16 | SASNVES | 17 | QQSRKDPST | 18 |

FIG. 15

VH and VL sequences of exemplary chimeric antibodies and humanized antibodies

| Antibody name | VH | Seq Id | VL | Seq Id |
|---|---|---|---|---|
| 3C4 | QVQLQQSGPELVKPGASVTISCKASDYSFTNYYIHWVKQRPGQGLEW IGWIYPGNVITKYHEKFKGKAILTADTSSTTAYMQLNSLTSEDSAVYF CARSRATGYFDYWGQGTTLTVSS | 19 | DIQMTQTTSSLSASLGDRVTISCRASQDIRNYLNWFQQ KPDGTIKLLIYYTSRLHSGVPSRFSGSGSGTDYSLTISNL EQEDMATYFCQQFNTLPWTFGGGTKLEIK | 20 |
| hz3C4. 7 | QVQLVQSGAEVKKPGASVKVSCKASDYSFTNYYIHWVRQAPGQGLE WMGWIYPGNVITKYHEKFKGRVTMTADTSTSTAYMELSSLRSEDTA VYYCARSRATGYFDYWGQGTLVTVSS | 21 | DIQMTQSPSSLSASVGDRVTITCRASQDIRNYLNWYQQ KPGKAPKLLIYYTSRLHSGVPSRFSGSGSGTDYTLTISS LQPEDFATYYCQQFNTLPWTFGGGTKVEIK | 22 |
| 72G8 | EVQLQQSGAELARPGASVMLSCKASGYTFTSYWMQWLKQRPGQGL EWIGAIYPGDGDTRYTQKFKGKATLTADKSSSTAYMQLSSLASEDSA VYYCVRDDGLYYAMDYWGQGTSVTVSS | 23 | QIVLTQSPTIMSASLGERVTMTCTASSSVSSSYLHWYQ QKPGSSPKLWIYSTSNLASGVPARFSGSGSGTSYSLTIS SMEAEDAATYYCHQYHRSPPTFGGGTKLEIK | 24 |
| 69B1 | QIQLVQSGPELKKPGETVKISCKASGYTFTDYSMNWVKQAPGKGLK WMGWLNTETGEPIYADDFKGRFVFSLETSASTAYLEINNLNDEDTAT YFCAREAIYYGTSYVLDYWGQGTSVTVSS | 25 | DIVLTQSPASLAVSLGQRATISCRASESVEYYGTNFMQ WYQQKPGQPPKLLIYSASNVESGVPARFSGSGSGTDFS LNIHPVEEDDIAVYFCQQSRKDPSTFGEGTNLEIK | 26 |
| hz69B 1.5 | QIQLVQSGSELKKPGASVKVSCKASGYTFTDYSMNWVRQAPGQGLE WMGWLNTETGEPIYADDFKGRFVFSLDTSVSTAYLQISSLKAEDTAV YFCAREAIYYGTSYVLDYWGQGTLVTVSS | 27 | DIVLTQSPASLAVSPGQRATITCRASESVEYYGTNFMQ WYQQKPGQPPKLLIYSASNVESGVPARFSGSGSGTDFT LTINPVEANDTANYYCQQSRKDPSTFGQGTKLEIK | 28 |
| hz69B 1.6 | QVQLVQSGAEVKKPGASVKVSCKASGYTFTDYSMNWVRQAPGQRL EWMGWLNTETGEPIYADDFKGRVTITRDTSASTAYMELSSLRSEDTA VYYCAREAIYYGTSYVLDYWGQGTLVTVSS | 29 | DIVLTQSPASLAVSPGQRATITCRASESVEYYGTNFMQ WYQQKPGQPPKLLIYSASNVESGVPARFSGSGSGTDFT LTINPVEANDTANYFCQQSRKDPSTFGQGTKLEIK | 30 |
| hz69B 1.11 | QVQLVQSGAEVKKPGASVKVSCKASGYTFTDYSMNWVRQAPGQRL EWMGWLNTETGEPIYADDFKGRVTITRDTSASTAYMELSSLRSEDTA VYYCAREAIYYGTSYVLDYWGQGTLVTVSS | 29 | DIVLTQSPASLAVSPGQRATITCRASESVEYYGTNFMQ WYQQKPGQPPKLLIYSASNVESGVPARFSGSGSGTDFT LTINPVEAQDTANYFCQQSRKDPSTFGQGTKLEIK | 31 |
| hz69B 1.20 | QIQLVQSGSELKKPGASVKVSCKASGYTFTDYSMNWVRQAPGQGLE WMGWLNTETGEPIYADDFKGRFVFSLDTSVSTAYLQISSLKAEDTAV YFCAREAIYYGTSYVLDYWGQGTLVTVSS | 64 | DIVLTQSPASLAVSPGQRATITCRASESVEYYGTNFMQ WYQQKPGQPPKLLIYSASNVESGVPARFSGSGSGTDFT LTINPVEAQDTANYFCQQSRKDPSTFGQGTKLEIK | 65 |

FIG. 16

EP 4 289 865 A1

## INTERNATIONAL SEARCH REPORT

| International application No. |
|---|
| **PCT/CN2022/074755** |

**A. CLASSIFICATION OF SUBJECT MATTER**

C07K 16/28(2006.01)i; C12N 15/13(2006.01)i; A61K 39/395(2006.01)i; A61K 47/68(2017.01)i; A61P 35/00(2006.01)i

According to International Patent Classification (IPC) or to both national classification and IPC

**B. FIELDS SEARCHED**

Minimum documentation searched (classification system followed by classification symbols)

C07K; C12N; A61K; A61P

Documentation searched other than minimum documentation to the extent that such documents are included in the fields searched

Electronic data base consulted during the international search (name of data base and, where practicable, search terms used)

VEN, CNABS, CNTXT, EPTXT, USTXT, WOTXT, ISI Web of Knowledge, CNKI, NCBI GENBANK: TNFRSF1B, TNF receptor superfamily member 1b, TNFRSF1B, TNF receptor 2, TNFR2, antibody, 信达生物, II型肿瘤坏死因子受体, 肿瘤坏死因子受体2, 抗体, 人源化, SEQ ID NOs: 19-30, 64-65

**C. DOCUMENTS CONSIDERED TO BE RELEVANT**

| Category* | Citation of document, with indication, where appropriate, of the relevant passages | Relevant to claim No. |
|---|---|---|
| A | WO 2018213064 A1 (HE UNITED STATES OF AMERICA, AS REPRESENTED BY THE SECRETARY, DEPARTMENT OF HEALTH AND HUMAN SERVICE) 22 November 2018 (2018-11-22)<br>claims 1, 6-7, 15, 23, 32-33, 35-36, and 43; and description, p. 39 | 1-21 |
| A | TORREY, H. et al. "A novel TNFR2 agonist antibody expands highly potent regulatory T cells"<br>*SCIENCE SIGNALING*, Vol. 13, 08 December 2020 (2020-12-08),<br>abstract; p. 8, right column, last paragraph to p. 9, left column, second paragraph | 1-21 |
| A | WO 2016187068 A1 (THE GENERAL HOSPITAL CORPORATION) 24 November 2016 (2016-11-24)<br>entire document | 1-21 |
| A | WO 2017220711 A1 (UNIVERSITE PARIS EST CRETEIL VAL DE MARNE et al.) 28 December 2017 (2017-12-28)<br>entire document | 1-21 |
| A | WO 2017040312 A1 (THE GENERAL HOSPITAL CORPORATION) 09 March 2017 (2017-03-09)<br>entire document | 1-21 |

☑ Further documents are listed in the continuation of Box C.     ☑ See patent family annex.

\* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **25 March 2022** | **26 April 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

| International application No. |
| --- |
| **PCT/CN2022/074755** |

**C.     DOCUMENTS CONSIDERED TO BE RELEVANT**

| Category* | Citation of document, with indication, where appropriate, of the relevant passages | Relevant to claim No. |
| --- | --- | --- |
| A | TORREY, H. et al. "Targeted killing of TNFR2-expressing tumor cells and Tregs by TNFR2 antagonistic antibodies in advanced Sézary syndrome" *LEUKEMIA*, Vol. 33, 24 October 2018 (2018-10-24), 1206-1218 | 1-21 |

Form PCT/ISA/210 (second sheet) (January 2015)

## INTERNATIONAL SEARCH REPORT

International application No.

**PCT/CN2022/074755**

**Box No. I**     **Nucleotide and/or amino acid sequence(s) (Continuation of item 1.c of the first sheet)**

1.  With regard to any nucleotide and/or amino acid sequence disclosed in the international application, the international search was carried out on the basis of a sequence listing:

    a.  ☑ forming part of the international application as filed:

        ☑ in the form of an Annex C/ST.25 text file.

        ☐ on paper or in the form of an image file.

    b.  ☐ furnished together with the international application under PCT Rule 13*ter*.1(a) for the purposes of international search only in the form of an Annex C/ST.25 text file.

    c.  ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2.  ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3.  Additional comments:

Form PCT/ISA/210 (continuation of first sheet) (January 2015)

**INTERNATIONAL SEARCH REPORT**

International application No.

**PCT/CN2022/074755**

| Box No. II | Observations where certain claims were found unsearchable (Continuation of item 2 of first sheet) |
|---|---|

This international search report has not been established in respect of certain claims under Article 17(2)(a) for the following reasons:

1. ☑ Claims Nos.: **16-17**
   because they relate to subject matter not required to be searched by this Authority, namely:

   [1]  Claim 16 relates to a use of an antibody in vivo, belonging to a disease treatment method; claim 17 relates to a disease treatment method, and does not comply with PCT Rule 39.1(iv). The present report is provided on the basis of amending the claims to be claims relating to a pharmaceutical use.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (January 2015)

INTERNATIONAL SEARCH REPORT
Information on patent family members

International application No.

**PCT/CN2022/074755**

| Patent document cited in search report | | | Publication date (day/month/year) | Patent family member(s) | | | Publication date (day/month/year) |
|---|---|---|---|---|---|---|---|
| WO | 2018213064 | A1 | 22 November 2018 | CA | 3059472 | A1 | 22 November 2018 |
| | | | | WO | 2018213064 | A8 | 14 November 2019 |
| | | | | US | 2020230173 | A1 | 23 July 2020 |
| | | | | AU | 2018268970 | A1 | 24 October 2019 |
| | | | | EP | 3625256 | A1 | 25 March 2020 |
| WO | 2016187068 | A1 | 24 November 2016 | US | 2021317221 | A1 | 14 October 2021 |
| | | | | CA | 2985816 | A1 | 24 November 2016 |
| | | | | HK | 1253012 | A1 | 06 June 2019 |
| | | | | AU | 2016263198 | A1 | 23 November 2017 |
| | | | | EP | 3294773 | A1 | 21 March 2018 |
| | | | | EP | 3447075 | A2 | 27 February 2019 |
| | | | | EP | 3447075 | A3 | 29 May 2019 |
| | | | | US | 2018194850 | A1 | 12 July 2018 |
| | | | | US | 10906982 | B2 | 02 February 2021 |
| | | | | CN | 107849142 | A | 27 March 2018 |
| WO | 2017220711 | A1 | 28 December 2017 | EP | 3475301 | A1 | 01 May 2019 |
| | | | | US | 2019330359 | A1 | 31 October 2019 |
| WO | 2017040312 | A1 | 09 March 2017 | EP | 3340999 | A1 | 04 July 2018 |
| | | | | EP | 3340999 | A4 | 12 June 2019 |
| | | | | US | 2019135929 | A1 | 09 May 2019 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2017083525 A
- US 5500362 A
- US 5821337 A
- US 6194551 B
- WO 9951642 A
- US 5677425 A
- EP 0154316 A
- EP 0401384 A
- US 5648237 A
- US 5789199 A
- US 5840523 A
- WO 2009080251 A
- WO 2009080252 A
- WO 2009080253 A
- WO 2010145793 A
- US 5208020 A
- WO 05103081 A
- WO 2015153513 A
- US 4737456 A

### Non-patent literature cited in the description

- Fundamental Immunology. Raven Press, 1989
- Fundamental Immunology. Raven Press, 1993
- Antibody Drug Research and Application,. People's Medical Publishing House, 2013
- HOLLINGER et al. *PNAS USA,* 1993, vol. 90, 6444-6448
- HUDSON et al. *Nat. Med.,* 2003, vol. 9, 129-134
- FLATMAN, S et al. *J. Chrom. B,* 2007, vol. 848, 79-87
- HARBOR ; LANE. Antibodies. Cold Spring Harbor Press
- KABAT, E.A et al. Sequences of Proteins of Immunological Interest. Public Health Service, National Institutes of Health, 1991
- HELLSTROM, I et al. *Proc. Nat'l Acad. Sci. USA,* 1986, vol. 83, 7059-7063
- HELLSTROM, I et al. *Proc. Nat'l Acad. Sci. USA,* 1985, vol. 82, 1499-1502
- BRUGGEMANN, M et al. *J. Exp. Med.,* 1987, vol. 166, 1351-1361
- CLYNES, R et al. *Proc. Nat'l Acad. Sci. USA,* 1998, vol. 95, 652-656
- LISZEWSKI ; ATKINSON. Fundamental immunology. Raven Press, 1993, 917-940
- RICHARDS et al. *Mol. Cancer Ther,* 2008, vol. 7 (8), 2517-2527
- KABAT et al. Sequences of Proteins of Immunological Interest. Public Health Service, National Institutes of Health, 1991
- CHOTHIA ; LESK. *J. Mol. Biol.,* 1987, vol. 196, 901-917
- PORTOLANO, S et al. *J. Immunol.,* 1993, vol. 150, 880-887
- CLACKSON, T. et al. *Nature,* 1991, vol. 352, 624-628
- IDUSOGIE, E. E. et al. *J. Immunol.,* 2000, vol. 164, 4178-4184
- SHIELDS, R. L. et al. *J. Biol. Chem.,* 2001, vol. 276, 6591-6604
- CHARLTON. Methods in Molecular Biology. Humana Press, 2003, vol. 248, 245-254
- GERNGROSS. *Nat. Biotech.,* 2004, vol. 22, 1409-1414
- LI et al. *Nat. Biotech,* 2006, vol. 24, 210-215
- GRAHAM et al. *J. Gen Virol,* 1977, vol. 36, 59
- URLAUB et al. *Proc. Natl. Acad. Sci. USA,* 1980, vol. 77, 216
- YAZAKI ; WU. Methods in Molecular Biology. Humana Press, 2003, vol. 248, 255-268
- CHARI et al. *Cancer Research,* 1992, vol. 52, 127-131
- Handbook of Pharmaceutical Excipients. Pharmaceutical Press
- Remington's Pharmaceutical Sciences. 1980
- ESTEP, P et al. High throughput solution based measurement of antibody-antigen affinity and epitope binning. *MAbs,* 2013, vol. 5 (2), 270-278